# EUROPÄISCHE PATENTANMELDUNG

(11) **EP 4 198 137 A1**
(43) Veröffentlichungstag der Anmeldung: **21.06.2023**
(21) Anmeldenummer: 21215759.8
(22) Anmeldetag: 17.12.2021
(51) Int. Cl.: C12P 13/00, C12N 1/20, C12N 9/10, C12N 9/12, C12N 9/88, C12N 9/92, C12N 15/52

(54) **MISCHUNGEN VON GLUCOSE UND XYLOSE ZUR FERMENTATIVEN HERSTELLUNG VON ORTHO-AMINOBENZOESÄURE**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft die Herstellung von ortho-Aminobenzoesäure mittels mikrobieller Fermentation, wobei Mischungen von Glucose und Xylose als fermentierbare Substrate verwendet werden.

## Beschreibung

Die vorliegende Erfindung betrifft die Herstellung von ortho-Aminobenzoesäure mittels mikrobieller Fermentation, wobei Mischungen von Glucose und Xylose als fermentierbare Substrate verwendet werden.

Die Herstellung von ortho-Aminobenzoesäure (oAB) mit Hilfe einer Reihe von genetisch veränderten Mikroorganismen aus dem Stand der Technik bekannt, z.B. aus Balderas-Hernandez et al. (2009) "Metabolic engineering for improving anthranilate synthesis from glucose in Escherichia coli", WO 2015/124687 und WO 2017/102853.

In all diesen Veröffentlichungen wurde die Verwendung von Glucose beschrieben. Einzelne Veröffentlichungen, so z.B. WO 2017/102853, beschreiben auch die Verwendung weiterer Kohlenstoffquellen. Die Vorteile der Kombination von Glucose und Xylose wurden aber nicht offenbart.

Labib et al. (2021) "Metabolic engineering for microbial production of protocatechuate with Corynebacterium glutamicum", Biotechnology and Bioengineering, 118: 4414-4427, beschreiben ein Verfahren, bei dem auf Grundlage von Glucose und Xylose Protocatechusäure fermentativ hergestellt wird. Hierbei wurde ein Stamm verwendet, bei dem Glucose nur in den Bau- und Erhaltungsstoffwechsel einging und die Produktbildung ausschließlich auf Xylose basierte. Die Kohlenstoffausbeute des dort beschriebenen Verfahrens ist aber gering. Von einem mol Kohlenstoff des von den Zellen verbrauchten Gesamtzuckers (Glucose und Xylose) ging nur 0,12 mol in das dort produzierte Produkt ein.

In der der vorliegenden Patentanmeldung zugrunde liegenden Studie wurde überraschend gefunden, dass eine Kombination von Glucose und Xylose in bestimmten Mengenverhältnissen zu einer besonders effizienten Umsetzung der Zucker zu ortho-Aminobenzoesäure (oAB) führt. Hierbei wurden um ein Drittel höhere Kohlenstoffausbeuten erreicht, als für ein ähnliches Verfahren zur Herstellung von Protocatechusäure berichtet.

Diese Erfindung wird in den Patentansprüchen und nachfolgend in der Beschreibung näher definiert.

In einer ersten Ausführungsform betrifft die vorliegende Erfindung ein Verfahren, enthaltend den Schritt der Kultivierung einer oder mehrerer mikrobieller Zellen, die Glucose und Xylose zu oAB umsetzen können, in einem Nährmedium, das ein Gemisch von Glucose und Xylose mit einem Glucoseanteil zwischen 5 Gew.-% und 86 Gew.-% und einem Xyloseanteil zwischen 95 Gew.-% und 14 Gew.-% enthält, wobei sich die Anteile von Glucose und Xylose zu 100 % addieren und wobei oAB produziert wird.

Die Inkubation wenigstens einer mikrobiellen Zelle im erfindungsgemäßen Nährmedium führt unter geeigneten Inkubationsbedingungen dazu, dass diese Zelle die im Nährmedium enthaltenen Zucker, bevorzugt die Glucose und die Xylose, besonders bevorzugt die Xylose zu oAB umsetzt. In einer bevorzugten Ausführungsform der vorliegende Erfindung sind die Stoffwechselwege in der mikrobiellen Zelle so getrennt, dass die Glucose ausschließlich zur Bildung von oAB verwendet wird, während das Wachstum auf Xylose stattfindet. Eine Produktbildung auch aus Xylose ist hierbei nicht ausgeschlossen.

Erfindungsgemäß werden pro Gramm Glucose und Xylose, das während der Inkubation von der wenigstens einen mikrobiellen Zelle verbraucht wird, wenigstens 0,09 Gramm oAB gebildet. Dies entspricht einer Kohlenstoffausbeute von wenigstens 0,138 mol Kohlenstoff in Form von oAB pro verbrauchtem mol Kohlenstoff in Glucose und Xylose. Unter besonders günstigen Bedingungen können diese Werte bei wenigstens 0,1 g oAB pro g verbrauchter Glucose und Xylose liegen, was einer Kohlenstoffausbeute von wenigstens 0,153 mol Kohlenstoff in oAB pro mol Kohlenstoff in Glucose und Xylose entspricht.

Der Begriff oAB bezeichnet die ortho-Aminobenzoesäure, auch unter der Bezeichnung "Anthranilsäure" bekannt. Da diese Verbindung sowohl über eine Carboxylgruppe mit sauren Eigenschaften als auch über eine Aminogruppe mit basischen Eigenschaften verfügt, ist die Ladung des Moleküls abhängig vom pH. Im Rahmen der vorliegenden Anmeldung bezeichnet der Begriff "ortho-Aminobenzoesäure" das Molekül unabhängig davon, ob es eine positive, eine negative oder gar keine Nettoladung hat.

### Nährmedium

Nährmedien, die zur Kultivierung mikrobieller Zellen, insbesondere von *Corynebacterium,* geeignet sind, sind aus dem Stand der Technik bekannt. Geeignete Nährmedien enthalten wenigstens einen Puffer zur Regulation des pH, für den verwendeten Mikroorganismus verwertbare Quellen anorganischer Nährstoffe, insbesondere Stickstoff, Schwefel und Phosphor sowie die vom eingesetzten Organismus benötigten Spurenelemente. Je nach Mikroorganismus kann der Zusatz von Vitaminen und/oder komplexen Medienbestandteilen wie z.B. Pepton oder Hefeextrakt sinnvoll sein. Besonders geeignete Nährmedien sind in den in dieser Anmeldung enthaltenen Ausführungsbeispielen beschrieben.

Das Nährmedium enthält obligatorisch Glucose und Xylose, wobei der Glucoseanteil zwischen 5 Gew.-% und 86 Gew.-% und der Xyloseanteil zwischen 95 Gew.-% und 14 Gew.-% liegt, und sich die Anteile von Glucose und Xylose zu 100 % addieren, als Energie- und Kohlenstoffquelle für den Mikroorganismus.

Diese dient sowohl dem Aufbau von Biomasse, als auch der Bildung von oAB. Die Anwesenheit weiterer Energie- und Kohlenstoffquellen ist dabei nicht ausgeschlossen. Es ist aber bevorzugt, dass der Massenanteil der Summe von Xylose und Glucose an der Gesamtmenge der im Nährmedium vorliegenden Zucker wenigstens 70 Gew.-%, bevorzugt wenigstens 80 Gew.-% und besonders bevorzugt wenigstens 90 Gew.-% beträgt. In einer bevorzugten Ausführungsform der vorliegenden Erfindung liegt im Nährmedium unter Einhaltung der oben definierten Gesamtanteile von Glucose und Xylose wenigstens ein Zucker ausgewählt aus der Gruppe bestehend aus Galactose, Arabinose, Cellobiose, Maltose und Fructose vor. Unabhängig von der Anwesenheit eines der vorgenannten Zucker kann das Nährmedium in dieser Ausführungsform auch Milchsäure und/oder Essigsäure enthalten.

In einer besonders bevorzugten Ausführungsform der vorliegenden Erfindung beträgt der Massenanteil der Summe von Xylose und Glucose an der Gesamtmenge aller im Nährmedium vorliegenden für den Mikroorganismus nutzbaren Energie- und Kohlenstoffquellen wenigstens 70 Gew.-%, bevorzugt wenigstens 80 Gew.-% und besonders bevorzugt wenigstens 90 Gew.-%. Die Begriffe "Energiequelle" und "Kohlenstoffquelle" sind dem Fachmann bekannt. Sie bezeichnen im Kontext von heterotrophen Mikroorganismen solche organischen Kohlenstoffverbindungen, aus denen der betreffende Mikroorganismus entweder die für seinen Stoffwechsel benötigte Energie gewinnen kann oder die er zum Aufbau von Biomasse nutzen kann.

Der Anteil an Glucose am Gemisch von Glucose und Xylose beträgt bevorzugt 5 Gew.-% bis 86 Gew.-%, stärker bevorzugt 8 Gew.-% bis 86 Gew.-%, noch stärker bevorzugt 12 Gew.-% bis 86 Gew.-% und am stärksten bevorzugt 16 Gew.-% bis 86 Gew.-%, wobei Xylose den jeweils zu 100 Gew.-% fehlenden Anteil beiträgt.

Bei der Ausbeute wird erfindungsgemäß zwischen zwei Werten unterschieden, der Substratausbeute und der Prozessausbeute. Bei der Substratausbeute wird die Menge der erzeugten oAB auf die Menge der während der Kultivierung von den mikrobiellen Zellen verbrauchten Glucose und Xylose bezogen. Restmengen an Glucose und Xylose, die zwar zum Nährmedium zugegeben wurden, am Ende der Kultivierung aber noch im Nährmedium anwesend sind, bleiben unberücksichtigt. Dies kann in Verfahrensvarianten, bei denen das Nährmedium nach Abtrennung der oAB erneut für eine Fermentation verwendet wird, vorteilhaft sein. Bei der Prozessausbeute wird die Menge der erzeugten oAB auf die Menge der während der Kultivierung dem Nährmedium zugesetzten Glucose und Xylose bezogen, unabhängig davon, ob diese Substrate vollständig umgesetzt wurden oder noch als Restmengen im Nährmedium vorliegen. Vereinfacht gesagt werden bei der Prozessausbeute von den mikrobiellen Zellen am Ende der Kultivierung nicht verbrauchte Mengen an Glucose und Xylose als Verlust verbucht.

Zur Erzielung einer möglichst hohen Prozessausbeute hat es sich als vorteilhaft erwiesen, wenn der Anteil an Glucose am Gemisch von Glucose und Xylose 40 Gew.-% bis 86 Gew.-%, bevorzugt 50 Gew.-% bis 86 Gew.-%, stärker bevorzugt 60 Gew.-% bis 86 Gew.-% und noch stärker bevorzugt 63 Gew.-% bis 86 Gew.-% beträgt, wobei Xylose den jeweils zu 100 Gew.-% fehlenden Anteil beiträgt.

### Kultivierung

Der Begriff "Kultivierung" bezeichnet die Inkubation einer oder mehrerer mikrobieller Zellen in dem oben beschriebenen Nährmedium unter Bedingungen, welche die Stoffwechselaktivität der Zellen ermöglichen. Dies bedeutet insbesondere die Einstellung und Aufrechterhaltung eines geeigneten pH-Wertes, einer geeigneten Sauerstoffsättigung, einer geeigneten Temperatur und einer geeigneten Osmolalität. Der Fachmann kann Kultivierungsbedingungen, die für den jeweiligen Mikroorganismus geeignet sind, anhand seiner Fachkenntnisse und der allgemein verfügbaren Literatur auswählen. Die Kultivierung von *Corynebacterium,* insbesondere *Corynebacterium glutamicum* erfolgt vorzugsweise bei einem pH zwischen 6 und 8, bei einer Temperatur zwischen 25 °C und 40 °C sowie bei einer Osmolalität zwischen 400 und 2600 mOsmol/kg. Die Sauerstoffsättigung wird vorzugsweise möglichst nahe an dem unter Normaldruck und Normalatmosphäre erreichbaren Maximum gehalten.

Die metabolische Aktivität besteht vorzugsweise in der Produktion von oAB und/oder dem Aufbau von Biomasse, besonders bevorzugt in der Produktion von oAB. Ein geeigneter Parameter, der die kontinuierliche Überwachung der metabolischen Aktivität zur Prozesssteuerung erlaubt, ist auch die Sauerstoffaufnahmerate. Diese kann, z.B. durch Sauerstoffelektroden, kontinuierlich erfolgen und liefert ohne zeitlichen Verzug Messergebnisse, die zur Anpassung der Kultivierungsbedingungen verwendet werden können.

Der Vorgang der Inkubation wenigstens einer mikrobiellen Zelle in einem oben beschriebenen Nährmedium unter Bedingungen, die die Bildung von oAB durch die mikrobiellen Zellen ermöglicht, wird nachfolgend auch als "mikrobielle Fermentation" bezeichnet.

### Mikrobielle Zelle

Bei der mikrobiellen Zelle handelt es sich vorzugsweise um eine heterotrophe Bakterienzelle, stärker bevorzugt ein Bakterium aus dem Genus *Corynebacterium* und besonders bevorzugt *Corynebacterium glutamicum.*

In einer bevorzugten Ausführungsform der vorliegenden Erfindung ist die mikrobielle Zelle ein *Corynebacterium,* bevorzugt *Corynebacterium glutamicum,*

Es hat sich überraschend gezeigt, dass ein Stamm mit derartigen genetischen Modifikationen Glucose und Xylose mit besonders hoher Kohlenstoffausbeute zu oAB umsetzt. Deswegen betrifft die vorliegende Anmeldung in einer weiteren Ausführungsform einen Stamm des Genus *Corynebacterium,* bevorzugt *Corynebacterium glutamicum* und besonders bevorzugt *Corynebacterium glutamicum* ATCC13032, der vom Wildtyp wenigstens in folgenden Merkmalen abweicht:
(i) Eine gegenüber dem jeweiligen Wildtyp reduzierte Aktivität der Anthranilat-Phosphoribosyltransferase, wobei aber eine Restaktivät vorhanden sein muss. Die Restaktivität liegt vorzugsweise zwischen 10 % und 60 %, stärker bevorzugt zwischen 20 % und 50 % der in *C. glutamicum* ATCC13032 nativ vorhandenen Aktivität. Dies wird vorzugsweise durch eine gegenüber dem Wildtyp reduzierte Expression des Gens für die Anthranilat-Phosphoribosyltransferase (trpD) erreicht, wobei die Expression aber nicht vollständig unterbunden ist. Dies erfolgt bevorzugt durch Einsatz einer Promotorsequenz, die gegenüber der endogen vorhandenen Promotorsequenz eine geringere Transkriptionsaktivität aufweist oder durch eine Modifikation des Abstands der Ribosomenbindestelle zum Start-Codon des trpD-Gens oder durch eine Veränderung des Start-Codons selbst. In einer bevorzugten Ausführungsform der vorliegenden Erfindung erfolgt die Reduzierung der Aktivität der Anthranilat-Phosphoribosyltransferase durch Deletion oder Inaktivierung des Gens für die endogene Anthranilat-Phosphoribosyltransferase (trpD) und den Ersatz dieses Gens durch ein Gen für eine Anthranilat-Phosphoribosyltransferase mit einer veränderten ribosomalen Bindestelle und optional einem veränderten Start-Codon wie in SEQ ID NO.1 oder 2, bevorzugt SEQ ID NO. 2 definiert. Die Aminosäuresequenz der Anthranilat-Phosphoribosyltransferase entspricht vorzugsweise der endogenen Anthranilat-Phosphoribosyltransferase, besonders bevorzugt wird sie durch SEQ ID NO. 3 oder eine Variante davon definiert.
(ii) Deletion oder Inaktivierung des Gens, das für die Phosphoenolpyruvat-Carboxylase wie in SEQ ID NO. 4 definiert kodiert. Dies kann auf alle dem Fachmann geläufigen Weisen geschehen, vorzugsweise durch Deletion des Gens oder einer Teilsequenz davon, durch Einführung wenigstens eines Stop-Codons oder durch Deletion oder Inaktivierung der Promotorsequenz. Besonders bevorzugt ist die Deletion wenigstens eines Teils der proteinkodierenden Sequenz des Gens (SEQ ID NO. 5).
(iii) Erhöhte Aktivität der Shikimat-Kinase. Dies erfolgt vorzugsweise durch erhöhte Expression eines entsprechenden Enzyms. In einer Ausführungsform der vorliegenden Erfindung erfolgt die Steigerung der Aktivität durch erhöhte Expression des Gens für die endogen vorhandene Shikimat-Kinase wie in SEQ ID NO. 6 definiert oder einer Variante davon. In einer anderen bevorzugten Ausführungsform erfolgt dies durch Expression einer exogenen Shikimat-Kinase, bevorzugt wie in SEQ ID NO. 7 definiert oder einer Variante davon. Eine verstärkte Expression eines Genes kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Ein besonders bevorzugter Promotor für die Expression von Fremdgenen oder die verstärkte Expression endogener Gene ist ptuf wie in SEQ ID NO. 8 definiert.
(iv) Erhöhte Aktivität der 3-Phosphoshikimat-1-carboxyvinyltransferase und Chorismat-Synthase. Bevorzugt haben diese Enzyme eine Aminosäuresequenz wie in SEQ ID NO 9 oder einer Variante davon und SEQ ID NO. 10 oder einer Variante davon definiert. Dies erfolgt bevorzugt durch die Einführung zusätzlicher Kopien der für diese Enzyme kodierenden Gene in den Mikroorganismus. Bevorzugt wird der ptuf-Promotor zur Steuerung der Expression verwendet.
(v) Anwesenheit einer 3-Desoxyarabinoheptulosanat-7-phosphat-Synthase (DAHP-Synthase), die "feedback-resistent" ist, d.h. nicht durch ihr Produkt oder durch ein aus dem Produkt entstehendes Produkt gehemmt wird. Bevorzugt ist ein Enzym mit der in SEQ ID NO. 11 definierten Aminosäuresequenz.
(vi) Erhöhte Aktivität der Xylose-Isomerase und der Xylulokinase. Diese erhöhte Aktivität erfolgt vorzugsweise durch verstärkte Expression der beiden Gene. Diese verstärkte Expression kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Erfindungsgemäß bevorzugt ist eine erhöhte Expression von Enzymen mit Aminosäuresequenzen wie in SEQ ID NO. 12 bzw. SEQ ID NO. 14 definiert oder Varianten davon.

Bezogen auf die Anthranilat-Phosphoribosyltransferase (SEQ ID NO. 3), Shikimat-Kinase (SEQ ID NO. 6 oder 7), 3-Phosphoshikimat-1-carboxyvinyltransferase (SEQ ID NO. 9), Chorismat-Synthase (SEQ ID NO. 10), Xylose-Isomerase (SEQ ID NO. 12) und die Xylulokinase (SEQ ID NO. 14) bedeutet "Variante" ein Enzym, das durch Hinzufügung, Deletion oder Austausch von bis zu 10 %, bevorzugt bis zu 5 %, der im jeweiligen Enzym enthaltenen Aminosäuren erhalten wird. Grundsätzlich können die vorgenannten Modifikationen an jeder beliebigen Stelle des Enzyms kontinuierlich oder diskontinuierlich erfolgen. Bevorzugt erfolgen sie aber nur am N-Terminus und/oder am C-Terminus des Polypeptids. Bei Substitutionen von Aminosäuren handelt es sich vorzugsweise um konservative Substitutionen, d.h. um solche, bei denen die veränderte Aminosäure einen Rest mit ähnlichen chemischen Eigenschaften besitzt wie die im unveränderten Enzym vorliegende Aminosäure. Es werden also besonders bevorzugt Aminosäuren mit basischen Resten gegen solche mit basischen Resten, Aminosäuren mit sauren Resten gegen solche mit ebenfalls sauren Resten, Aminosäuren mit polaren Resten gegen solche mit polaren Resten und Aminosäuren mit unpolaren gegen solche mit unpolaren Resten ausgetauscht. Bevorzugt Die spezifische Enzymaktivität einer Variante liegt vorzugsweise wenigstens bei 80 % spezifischen Aktivität des unveränderten Enzyms. Enzymtests zum Nachweis der Aktivität der vorgenannten Enzyme kann der Fachmann der Literatur entnehmen.

Bezogen auf die DAHP-Synthase (SEQ ID NO. 11) bedeutet "Variante" ein Enzym, das durch Hinzufügung, Deletion oder Austausch von bis zu 5 %, bevorzugt bis zu 2 %, der im jeweiligen Enzym enthaltenen Aminosäuren erhalten wird, mit der Maßgabe, dass die Positionen 76 und 211 unverändert bleiben. Es ist weiterhin bevorzugt, dass zusätzlich auch die Positionen 10, 13, 147, 148, 150, 151, 179, 209, 211 und 212 unverändert bleiben. In einer stärker bevorzugten Ausführungsform der vorliegenden Erfindung sind in der Variante zusätzlich auch die Positionen 144, 175 und 215 unverändert. In einer noch stärker bevorzugten Ausführungsform der vorliegenden Erfindung sind zusätzlich zu den vorgenannten Positionen auch die Positionen 92, 97, 165, 186 und 268 unverändert. Der Fachmann versteht, dass sich die vorgenannten Positionen entsprechend verschieben, wenn Aminosäuren deletiert oder eingefügt werden. Grundsätzlich können die vorgenannten Modifikationen an jeder beliebigen Stelle des Enzyms kontinuierlich oder diskontinuierlich erfolgen. Bevorzugt erfolgen sie aber nur am N-Terminus und/oder am C-Terminus des Polypeptids. Bei Substitutionen von Aminosäuren handelt es sich vorzugsweise um konservative Substitutionen, d.h. um solche, bei denen die veränderte Aminosäure einen Rest mit ähnlichen chemischen Eigenschaften besitzt wie die im unveränderten Enzym vorliegende Aminosäure. Es werden also besonders bevorzugt Aminosäuren mit basischen Resten gegen solche mit basischen Resten, Aminosäuren mit sauren Resten gegen solche mit ebenfalls sauren Resten, Aminosäuren mit polaren Resten gegen solche mit polaren Resten und Aminosäuren mit unpolaren gegen solche mit unpolaren Resten ausgetauscht. Es ist besonders bevorzugt, dass auch die Variante der DAHP-Synthase eine entsprechende Enzymaktivität besitzt. Besonders bevorzugt liegt die spezifische Enzymaktivität der Variante bei wenigstens bei 80 % spezifischen Aktivität der unveränderten DAHP-Synthase gemäß SEQ ID NO. 11.

In noch einer weiteren Ausführungsform betrifft die vorliegende Erfindung die Verwendung eines Nährmediums, das ein Gemisch von Glucose und Xylose mit einem Glucoseanteil zwischen 5 Gew.-% und 86 Gew.-% und einem Xyloseanteil zwischen 95 Gew.-% und 14 Gew.-% enthält, wobei sich die Anteile von Glucose und Xylose zu 100 % addieren, zur Herstellung von oAB durch eine mikrobielle Fermentation.

Alle weiter oben in dieser Anmeldung gegebenen Definitionen gelten auch für diese Ausführungsform.

In noch einer weiteren Ausführungsform betrifft die vorliegende Anmeldung eine Zelle des Genus *Corynebacterium,* bevorzugt *Corynebacterium glutamicum* und besonders bevorzugt *Corynebacterium glutamicum* ATCC13032, der vom Wildtyp wenigstens in folgenden Merkmalen abweicht:
(i) Eine gegenüber dem jeweiligen Wildtyp reduzierte Aktivität der Anthranilat-Phosphoribosyltransferase, wobei aber eine Restaktivät vorhanden sein muss. Die Restaktivität liegt vorzugsweise zwischen 10 % und 60 %, stärker bevorzugt zwischen 20 % und 50 % der in *C*. *glutamicum* ATCC13032 nativ vorhandenen Aktivität. Dies wird vorzugsweise durch eine gegenüber dem Wildtyp reduzierte Expression des Gens für die Anthranilat-Phosphoribosyltransferase (trpD) erreicht, wobei die Expression aber nicht vollständig unterbunden ist. Dies erfolgt bevorzugt durch Einsatz einer Promotorsequenz, die gegenüber der endogen vorhandenen Promotorsequenz eine geringere Transkriptionsaktivität aufweist oder durch eine Modifikation des Abstands der Ribosomenbindestelle zum Start-Codon des trpD-Gens oder durch eine Veränderung des Start-Codons selbst. In einer bevorzugten Ausführungsform der vorliegenden Erfindung erfolgt die Reduzierung der Aktivität der Anthranilat-Phosphoribosyltransferase durch Deletion oder Inaktivierung des Gens für die endogene Anthranilat-Phosphoribosyltransferase (trpD) und den Ersatz dieses Gens durch ein Gen für eine Anthranilat-Phosphoribosyltransferase mit einer veränderten ribosomalen Bindestelle und optional einem veränderten Start-Codon wie in SEQ ID NO.1 oder 2, bevorzugt SEQ ID NO. 2 definiert. Die Aminosäuresequenz der Anthranilat-Phosphoribosyltransferase entspricht vorzugsweise der endogenen Anthranilat-Phosphoribosyltransferase, besonders bevorzugt wird sie durch SEQ ID NO. 3 oder eine Variante davon definiert.
(ii) Deletion oder Inaktivierung des Gens, das für die Phosphoenolpyruvat-Carboxylase wie in SEQ ID NO. 4 definiert kodiert. Dies kann auf alle dem Fachmann geläufigen Weisen geschehen, vorzugsweise durch Deletion des Gens oder einer Teilsequenz davon, durch Einführung wenigstens eines Stop-Codons oder durch Deletion oder Inaktivierung der Promotorsequenz. Besonders bevorzugt ist die Deletion wenigstens eines Teils der proteinkodierenden Sequenz des Gens (SEQ ID NO. 5).
(iii) Erhöhte Aktivität der Shikimat-Kinase. Dies erfolgt vorzugsweise durch erhöhte Expression eines entsprechenden Enzyms. In einer Ausführungsform der vorliegenden Erfindung erfolgt die Steigerung der Aktivität durch erhöhte Expression des Gens für die endogen vorhandene Shikimat-Kinase wie in SEQ ID NO. 6 definiert oder einer Variante davon. In einer anderen bevorzugten Ausführungsform erfolgt dies durch Expression einer exogenen Shikimat-Kinase, bevorzugt wie in SEQ ID NO. 7 definiert oder einer Variante davon. Eine verstärkte Expression eines Genes kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Ein besonders bevorzugter Promotor für die Expression von Fremdgenen oder die verstärkte Expression endogener Gene ist ptuf wie in SEQ ID NO. 8 definiert.
(iv) Erhöhte Aktivität der 3-Phosphoshikimat-1-carboxyvinyltransferase und Chorismat-Synthase. Bevorzugt haben diese Enzyme eine Aminosäuresequenz wie in SEQ ID NO 9 oder einer Variante davon und SEQ ID NO. 10 oder einer Variante davon definiert. Dies erfolgt bevorzugt durch die Einführung zusätzlicher Kopien der für diese Enzyme kodierenden Gene in den Mikroorganismus. Bevorzugt wird der ptuf-Promotor zur Steuerung der Expression verwendet.
(v) Anwesenheit einer 3-Desoxyarabinoheptulosanat-7-phosphat-Synthase (DAHP-Synthase), die "feedback-resistent" ist, d.h. nicht durch ihr Produkt oder durch ein aus dem Produkt entstehendes Produkt gehemmt wird. Bevorzugt ist ein Enzym mit der in SEQ ID NO. 11 definierten Aminosäuresequenz.
(vi) Erhöhte Aktivität der Xylose-Isomerase und der Xylulokinase. Diese erhöhte Aktivität erfolgt vorzugsweise durch verstärkte Expression der beiden Gene. Diese verstärkte Expression kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Erfindungsgemäß bevorzugt ist eine erhöhte Expression von Enzymen mit Aminosäuresequenzen wie in SEQ ID NO. 12 bzw. SEQ ID NO. 14 definiert oder Varianten davon.

Alle Definitionen, die weiter oben in dieser Anmeldung gegeben wurden, gelten auch für diese Ausführungsform. Dies gilt insbesondere für die genetischen und metabolischen Eigenschaften der Zelle.

In noch einer weiteren Ausführungsform betrifft die vorliegende Erfindung die Verwendung einer mikrobiellen Zelle, die vom Wildtyp in den oben in dieser Anmeldung definierten Merkmalen abweicht, zur Herstellung von oAB durch eine mikrobielle Fermentation mit einem Gemisch von Glucose und Xylose als Energie- und Kohlenstoffquelle.

Alle Definitionen, die weiter oben in dieser Anmeldung gegeben wurden, gelten auch für diese Ausführungsform. Dies betrifft insbesondere die Kultivierungsbedingungen, die geeignete Konzentrationen und Mengenverhältnisse von Glucose und Xylose sowie die für die mikrobielle Fermentation eingesetzten mikrobiellen Zellen.

In noch einer weiteren Ausführungsform betrifft die vorliegende Erfindung eine Zusammensetzung enthaltend
a) mikrobielle Zellen, die Glucose und Xylose zu oAB umsetzen können;
b) ein Gemisch von Glucose und Xylose mit einem Glucoseanteil zwischen 5 Gew.-% und 86 Gew.-% und einem Xyloseanteil zwischen 95 Gew.-% und 14 Gew.-%, wobei sich die Anteile von Glucose und Xylose zu 100 % addieren; und
c) wenigstens eine Stickstoffquelle, wenigstens eine Phosphorquelle, wenigstens eine Schwefelquelle und Spurenelemente.

Alle weiter oben in dieser Anmeldung gegebenen Definitionen gelten auch für diese Ausführungsform.

Bei der erfindungsgemäßen Zusammensetzung handelt es sich um ein Nährmedium, in dem die mikrobiellen Zellen enthalten sind, so dass nur noch die oben beschriebenen Inkubationsbedingungen eingehalten werden müssen, damit die mikrobiellen Zellen die enthaltene Glucose und die Xylose zu oAB umsetzen.

Wenn die oben definierte Zusammensetzung bestimmungsgemäß genutzt wird, produzieren die darin enthaltenen mikrobiellen Zellen oAB. Deswegen enthält die Zusammensetzung in einer bevorzugten Ausführungsform der vorliegenden Erfindung zusätzlich oAB. Stärker bevorzugt enthält sie wenigstens 1,5 g/l oAB.

Die nachfolgenden Ausführungsbeispiele dienen nur dazu, die Erfindung zu illustrieren. Sie sollen den Schutzbereich der Patentansprüche in keiner Weise beschränken.

### Beispiele

Alle Experimente wurden mit dem Stamm dessen Erzeugung nachfolgend beschrieben ist.

Auf Grundlage des Bakteriums *Corynebacterium glutamicum* ATCC13032 wurde ein mikrobieller Stamm erzeugt, der in der Lage ist, Xylose als Kohlenstoffquelle zur Produktion von Anthranilsäure zu verwerten. Dazu wurde der Ausgangsstamm zunächst durch adaptive Laborevolution (ALE) an steigende Anthranilsäurekonzentrationen im Kulturüberstand angepasst. Im Folgenden wurde dieser durch gerichtete chromosomale Modifikationen zur Produktion von Anthranilsäure gebracht. Anschließend wurde der Stamm mit Genen des sogenannten Xylose-Isomerase-Wegs ausgestattet, da *C. glutamicum* ATCC13032 über keinen nativen Stoffwechselweg zur Verwertung von Xylose verfügt. Alle genetischen Modifikationen .d.h. chromosomale Deletionen und Integration von Genen, erfolgten durch doppelte homologe Rekombination unter Verwendung von entsprechenden pK19*mobsacB-*Derivaten (Schäfer et al., 1994: "Small mobilizable multi-purpose cloning vectors derived from the Escherichia coli plasmids pK18 and pK19: selection of defined deletions in the chromosome of Corynebacterium glutamicum." Gene 145(1):69-73. doi: 10.1016/0378-1119(94)90324-7)

Die Aktivität der Anthranilatphosphoribosyltransferase TrpD wurde herabgesetzt, indem zunächst das native *trp*D-Allel deletiert und durch ein Allel (genannt *trpD5*) mit GTG- statt ATG-Startcodon und Ribosomenbindestelle mit verringertem Abstand zum Startcodon ersetzt wurde (SEQ ID NO. 1).

Es wurde das Gen (SEQ ID NO. 5)deletiert, welches für eine bzw. die einzige Phosphoenolpyruvat-Carboxylase in *C. glutamicum* (SEQ ID NO. 4) kodiert.

Zur Verstärkung des Aromatenbiosynthesewegs wurde ein artifizielles, polycistronisches P_{tuf}*-aroLAC-*Operon (SEQ ID NO. 15), bestehend aus den Genen *aroL* (*b0388*) aus *Escherichia coli,* sowie *aroA (cg0873)* und *aroC* (*cg1829*) aus *C. glutamicum,* unter der Kontrolle des konstitutiven Promoters des Elongationsfaktors Tuf, strangabwärts von *cg2563* integriert. Zusätzlich wurde das Allel aroG^{new}, welches für eine feedbackresistente Variante der DAHP-Synthase (SEQ ID NO. 11) aus *E. coli* kodiert, strangabwärts von *cg3132* in das Genom des Stamms integriert.

Um den Stamm zu befähigen, Xylose in den nichtoxidativen Pentosephosphatweg einspeisen zu können, wurde ein synthetisches Konstrukt, bestehend aus einem codonoptimierten Xylose-Isomerase-Gen *xylA* (xcc1758 )aus *Xanthomonas campestris* pv. campestris (Aminosäuresequenz des Enzyms gemäß SEQ ID NO. 13), sowie dem Xylulokinase-Gen *xylB* (cg0147) aus *C*. *glutamicum* (Aminosäuresequenz des Enzyms gemäß SEQ ID NO. 14), jeweils unter der Kontrolle der P_{tuf}-Promotorsequenz ((SEQ ID NO. 8)) und gefolgt von einem *rrnB*-Terminator aus *E. coli* erzeugt. Das P_{tuf}-*xylA_{Xcc}-*P_{tuf}*-xylB_{Cg}-*T_{rrnB} genannte Konstrukt (SEQ ID NO. 16) wurde stromabwärts von *cg3344* in das Genom des Stammes integriert.

### Beispiel 1

Es wurden vergleichende Kultivierungen des oben genannten Stamms zur Produktion von ortho-Aminobenzoesäure ausgehend von zwei verschiedenen Zuckern in jeweils vier verschiedenen Verhältnissen zueinander durchgeführt. Dabei wurden die Hauptkulturmedien so hergestellt, dass die beiden Zucker _{D}-Glucose und _{D}-Xylose in Mengen wie in Tabelle 1 bezogen auf den Gesamtzuckergehalt von 20 g/L vorlagen.

**Tabelle 1: Übersicht über die Zusammensetzungen der zwei verschiedenen Zucker _{D}-Glucose und _{D}-Xylose in den Hauptkultivierungsmedien. Für die Medien 1-4 und damit jedes Zuckermengenverhältnis wurden je vier Replikate mit einem initialen Kultivierungsvolumen von je 50 mL angelegt.**

| | Gew.-% Zucker (Anteil bezogen auf den Gesamtzuckergehalt) | |
|---|---|---|
| Bezeichnung Medium | _{D}-Glucose | _{D}-Xylose |
| Medium 1 | 100 | 0 |
| Medium 2 | 64 | 36 |
| Medium 3 | 75 | 25 |
| Medium 4 | 88 | 12 |

Für jede Bedingung bzw. jedes Zuckermengenverhältnis aus Tabelle 1 wurden je vier Replikate angelegt. Die Kultivierungsbedingungen sind nachfolgend dargestellt (Tabelle 2).

**Tabelle 2: Übersicht über die Kultivierungsbedingungen der Vor- und Hauptkulturen. Für die Inkubation der zweiten Vorkulturen (je 25 mL) wurden Erlenmeyer-Kolben mit einem maximalen Fassungsvolumen von je 500 mL verwendet, für die Inkubation der Hauptkulturen (initial vor der ersten Probennahme je 50 mL) Erlenmeyer-Kolben mit einem maximalen Fassungsvolumen von je 1000 mL. Als Sterilbarriere dienten Baumwollstopfen.**

| Parameter | Einstellung | Bemerkung |
|---|---|---|
| Schüttelfrequenz (rpm) | 200 | - |
| Temperatur (°C) | 30 | - |
| Initiales Volumen pro Replikat (mL) | 25 | Im 500 mL-Erlenmeyerkolben, für die Vorkultur |
| Initiales Volumen pro Replikat (mL) | 50 | Im 1000 mL Erlenmeyerkolben, für die Hauptkultur |

### Verwendete Medien, deren Zusammensetzung und Herstellung

Wenn nicht anders beschrieben, werden alle Medien mit ddH₂O hergestellt und autoklaviert.

**Tabelle 3: Flüssige und feste Komplexmedien aus Brain-Heart-Infusion (BHI) zur Anzucht von Zellen.**

| **Medium** | **Zusammensetzung** | **Zugabe vor/nach Autoklav.** |
|---|---|---|
| **BHI-Broth** | 37 g/L BHI-Broth | Vor dem Autoklavieren |
| **BHI-Agarplatten** | 52 g/L BHI-Agar | Vor dem Autoklavieren |

**Tabelle 4: Flüssiges Minimalmedium mit Komplexbestandteilen für die Anzucht von Zellen in der Vorkultur. Die Mengen der Einwaage sind für 1 L fertiges CGXII Vorkulturmedium angegeben. Nachdem alle Reagenzien im Medium supplementiert wurden, werden die final angestrebten Konzentrationen im fertigen Medium erreicht.**

| **Medium** | **Zusammensetzung** | **Entspricht finaler Konzentration im fertigen Medium** | **Zugabe vor/nach Autoklav.** |
|---|---|---|---|
| **CGXII Vorkulturmedium** | 1 g KH₂PO₄ | 1 g/L | Ab hier alles vor dem Autoklavieren |
| | 1 g K₂HPO₄ | 1 g/L | hinzu. |
| | 10 g (NH₄)₂SO₄ | 10 g/L | |
| | 5 g Urea | 5 g/L | |
| | 62 g MOPS | 62 g/L | |
| | 5 g Hefeextrakt | 5 g/L | |
| | 1 mL CaCl₂-Stammlösung | 1 mL/L | |
| **In 800 mL ddH₂O lösen, pH mit KOH-Plättchen einstellen auf pH=7,0** - **7,5, mit ddH₂O auffüllen auf 940 mL und autoklavieren.** | | | |
| | 1,25 mL MgSO₄-Stammösung | 1,25 mL/L | Ab hier alles nach dem Autoklavieren hinzu: |
| | 1 mLSpurenelement-Stammlösung | 1 mL/L | |
| | 1 mL Biotin-Stammlösung | 1 mL/L | |
| | 33 mL _{D}-Glucose- ODER | 20 g/L _{D}-Glucose- ODER | |
| | 33 mL _{D}-Glucose und 16,5 mL _{D}-Xylose-Stammlösung | 20 g/L _{D}-Glucose und 10 g/L _{D}-Xylose | |
| | 23,75 mL Wasser ODER | - | |
| | 7,25 mL Wasser | | |

**Tabelle 5: Flüssiges Minimalmedium für die Hauptkultur. Die Mengen der Einwaage sind für 1 L fertiges CGXII Hauptkulturmedium angegeben. Nachdem alle Reagenzien im Medium supplementiert wurden, werden die final angestrebten Konzentrationen im fertigen Medium erreicht.**

| **Medium** | **Zusammensetzung** | **Entspricht finaler Konzentration im fertigen Medium** | **Zugabe vor/nach Autoklav.** |
|---|---|---|---|
| **CGXII Hauptkulturmedium** | 1 g KH₂PO₄ | 1 g/L | Ab hier alles vor dem Autoklavieren hinzu: |
| | 1 g K₂HPO₄ | 1 g/L | |
| | 10 g (NH₄)₂SO₄ | 10 g/L | |
| | 62 g MOPS | 62 g/L | |
| | 1 mL CaCl₂-Stammlösung | 1 mL/L | |
| **In 800 mL ddH₂O lösen, pH mit KOH-Plättchen einstellen auf pH=7,0 - 7,5, mit ddH₂O auffüllen auf 940 mL und autoklavieren.** | | | |
| | 1,25 mL MgSO₄-Stammlösung | 1,25 mL/L | Ab hier alles nach dem Autoklavieren: |
| | 1 mL Spurenelement-Stammlösung | 1 mL/L | |
| | 1 mL Biotin-Stammlösung | 1 mL/L | |
| | 33 mL _{D}-Glucose- ODER | 20 g/L _{D}-Glucose- ODER | |
| | 33 mL _{D}-Xylose-Stammlösung | 20 g/L _{D}-Xylose | |
| | 23,75 mL Wasser | - | |

**Tabelle 6: Übersicht zur Herstellung der 2 g/L Biotin-Stammlösung. Die Lösung wird nicht autoklaviert, sondern steril filtriert (0,2 µm). Die Lösung kann für 1 Monat bei 4 °C aufbewahrt werden.**

| **Medium** | **Zusammensetzung** | **Notiz** |
|---|---|---|
| **Biotin-Stammlösung** | 2 g/L Biotin | Lösung wird steril filtriert. |
| | | Während des Lösens wird unter Rühren der pH-Wert gemessen und dieser mit 1 M KOH auf pH=7,2 eingestellt. |

**Tabelle 7: Übersicht zur Herstellung der 600 g/L _{D}-Glucose-Stammlösung.**

| **Medium** | **Zusammensetzung** | **Notiz** |
|---|---|---|
| **_{D}-Glucose-Stammlösung** | 660 g _{D}-Glucose-Monohgydrat | Substanz wird in 554 g heißem ddH₂O gelöst. Gesamtgewicht von 1 L der fertigen Lösung: 1214 g. Zum vollständigen Lösen vor dem Autoklavieren kurz Aufkochen. |

**Tabelle 8: Übersicht zur Herstellung der 600 g/L _{D}-Xylose-Stammlösung.**

| **Medium** | **Zusammensetzung** | **Notiz** |
|---|---|---|
| **_{D}-Xylose-Stammlösung** | 600 g _{D}-Xylose | Substanz wird in 595 g heißem ddH₂O gelöst. Gesamtgewicht von 1 L der fertigen Lösung: 1195 g. Zum vollständigen Lösen vor dem Autoklavieren kurz Aufkochen. |

**Tabelle 9: Übersicht zur Herstellung der 10 g/L CaCl₂-Stammlösung.**

| **Medium** | **Zusammensetzung** | **Notiz** |
|---|---|---|
| **CaCl₂-Stammlösung** | 10 g/L CaCl₂ × 2 H₂O | Lösung muss nicht autoklaviert werden, da sie vor dem Autoklaviervorgang der CGXII-Medien supplementiert wird. |

**Tabelle 10: Übersicht zur Herstellung der 200 g/L MgSO₄-Stammlösung.**

| **Medium** | **Zusammensetzung** | **Notiz** |
|---|---|---|
| **MgSO₄-Stammlösung** | 200 g/L MgSO₄ × 7 H₂O | Lösung wird steril filtriert. |

**Tabelle 11: Übersicht zur Herstellung der 1000x Spurenelement-Lösung. Die Lösung wird nicht autoklaviert, sondern steril filtriert (0,2 µm). Diese Lösung ist 6 Monate bei 4 °C haltbar. Empfehlenswert aufgrund der geringen Einwaagen ist die Herstellung eines Ansatzes von 1 L.**

| **Medium** | **Zusammensetzung** | **Notiz** |
|---|---|---|
| **Spurenelement-Lösung** | 10 g/L MnSO₄ × H₂O | Lösung wird steril filtriert. |
| | 10 g/L FeSO₄ × 7 H₂O | |
| | 1 g/L ZnSO₄ × 7 H₂O | |
| | 0,2 g/L CuSO₄ × 5 H₂O | |
| | 0,02 g/L NiCl₂ × 6 H₂O | |
| In ddH₂O lösen und pH mit HCl einstellen auf pH=1. | | |

**Tabelle 12: Übersicht zur Herstellung von 1x Phosphatpuffer (PBS). Verwendet wurde der 10x PBS (Artikelnummer BP399-1) der Firma Fisher Scientific GmbH.**

| **Medium** | **Zusammensetzung** | **Notiz** |
|---|---|---|
| **1x Phosphatpuffer** | 10x PBS | 1:10 verdünnen mit ddH₂O, autoklavieren. |

### Verwendete Geräte

**Tabelle 13: Übersicht über die in dieser Studie untersuchten Parameter sowie die Geräte und Verfahren zu deren Untersuchung.**

| Parameter | Gerät | Beschreibung |
|---|---|---|
| Glucose-Konzentration | Cedex Bio Analyzer, Roche S/N 35163 | Assay Glucose Cedex Bio mit der Artikelnummer RD-06343732001 nach Herstelleranleitung verwendet |
| NH₃-Konzentration | Cedex Bio Analyzer, Roche S/N 35163 | Assay NH3 Cedex Bio mit der Artikelnummer RD-06343775001 nach Herstelleranleitung verwendet. |
| ortho-Aminobenzoesäure-Konzentration | HPLC, Agilent G7104C 1260 Flexible Pump S/N DEAGZ01520 G7167A 1260 Multisampler S/N DEAGY01567 G7116A 1260 Multicolumn Thermostat (MCT) S/N DEAEM05811 G7117C 1260 DAD HS S/N DEAEK06638 G7162A 1260 RID S/N DEAC904783 | Zur Trennung und Quantifizierung der ortho-Aminobenzoesäure im sterilfiltrierten Überstand der Proben wurden von der Firma Agilent eine Eclipse Plus C18 Säule (4,6 × 150 mm, 5 µm, S/N USUXB19935) und eine Zorbax Eclipse Plus C18 Vorsäulenkartusche (4,6 × 12 mm, 5 µm, 5 mm) verwendet. Die Detektion der ortho-Aminobenzoesäure erfolgte über den Diodenarray-Detektor (DAD). |
| _{D}-Xylose-Konzentration | HPLC, ROA HPLC, Agilent G7104C 1260 Flexible Pump S/N DEAGZ01520 G7167A 1260 Multisampler S/N DEAGY01567 G7116A 1260 Multicolumn Thermostat (MCT) S/N DEAEM05811 G7117C 1260 DAD HS S/N DEAEK06638 G7162A 1260 RID S/N DEAC904783 | Zur Trennung und Quantifizierung der _{D}-Xylose im sterilfiltrierten Überstand der Proben wurden von der Firma Phenomenex eine Rezex ROA-Organic Acid H+ 8 % Säule (300 × 7,8 mm, S/N H20-094694) und eine SecurityGuard Cartridge Carbo-H Vorsäulenkartusche (4 × 3,0 mm) verwendet. Die Detektion der _{D}-Xylose erfolgte über den Brechungsindexdetektor (RID). |
| Inkubation der Kulturen | Schüttelinkubator ISF1-X, Kuhner Shaker GmbH S/N 65004.660 und S/N 65004.659 | |
| pH-Wert | pH-Meter SevenCompact S210, Mettler Toledo S/N B921689454 pH-Elektrode InLab Expert Pro-ISM, Mettler Toledo S/N 30014096 | |
| Optische Dichte (OD₆₀₀) | CO8000 Cell Density Meter, WPA biowave S/N 80-3000-45-D1-FR-E 1113 | |
| Gewicht Biotrockenmasse | SARTORIUS CUBIS^{®} Semimikrowaage 225S, Sartorius S/N 38101395 | |

### Durchführung

Von Glycerinüberdauerungsformen der für die Kultivierung verwendeten mikrobiellen Kulturen wurde zur Generierung der Starterkulturen Zellmasse entnommen und diese zum Beimpfen einer BHI-Agarplatte verwendet. Die BHI-Agarplatten wurde bei 30 °C für 72 h inkubiert.

Die auf diese Weise angelegte Agarplattenkultur wurde zum Beimpfen einer BHI-Flüssigkultur verwendet. Dazu wurde den jeweiligen BHI-Agarplattenkulturen etwas Zellmasse entnommen und je 4 mL BHI-Flüssigmedium in einem Rundbodenröhrchen beimpft. Die Flüssigkulturen wurden etwa 7,5 h bei 30 °C und 200 rpm inkubiert (Vorkultur I).

Für die zweite Vorkultur wurden zwei Medien mit verschiedenen Kohlenstoffquellen verwendet. Dazu wurde CGXII Vorkulturmedium mit entweder 20 g/L _{D}-Glucose oder mit 20 g/L _{D}-Glucose und zusätzlich 10 g/L _{D}-Xylose hergestellt. Dazu wurden je 23 mL des entsprechenden Vorkulturmediums in einen 500 mL-Erlenmeyerkolben überführt, zu beiden Ansätzen je 2 mL der BHI-Flüssigvorkultur I gegeben und diese beiden Schüttelkolbenvorkulturen bei 200 rpm und 30 °C für 17 h inkubiert.

Die Vorkultur mit _{D}-Glucose als einziger Energie- und Kohlenstoffquellewurde für die Inokulation der Hauptkulturen mit Medium 1 verwendet. Ausgehend von der Vorkultur mit 20 g/L _{D}-Glucose und zusätzlich 10 g/L _{D}-Xylose wurden drei verschiedene Hauptkulturen (Medien 2-4) beimpft.

Nach erfolgter Inkubation der zweiten Vorkultur im Schüttelkolben wurde die optische Dichte der Kulturen gemessen. Abhängig von der erreichten Zelldichte wurde ein Anteil dieser zweiten Vorkulturen entnommen, die Zellen durch Zentrifugation pelletiert, in sterilem Phosphatpuffer (einfach-konzentriert) gewaschen und in je 50 mL des entsprechenden Hauptkulturmediums (Medien 1-4) resuspendiert.

**Tabelle 14: Messung der optischen Dichte (OD₆₀₀) nach erfolgter Inkubation der zweiten Vorkultur im Schüttelkolben. Anhand dessen wurde berechnet, wie viel Volumen der zweiten Vorkultur nötig ist, um je 50 mL Hauptkultur mit einer initialen OD₆₀₀ von 1 zu beimpfen. Pro Medium wurden vier Replikate angelegt. Da drei xylosehaltige Medien (Medien 2-4) getestet werden sollten, wurden von der Vorkultur mit 20 g/L D-Glucose und 10 g/L D-Xylose insgesamt 12 Aliquots für die Inokulation entnommen.**

| | | |
|---|---|---|
| | OD₆₀₀(-) | Benötigtes Volumen |
| mit 20 g/L _{D}-Glucose | 30 | 4 × 1,7 mL für Medium 1 |
| mit 20 g/L _{D}-Glucose und 10 g/L _{D}-Xylose | 32 | 4 × 1,6 mL für Medium 2 |
| | | 4 × 1,6 mL für Medium 3 |
| | | 4 × 1,6 mL für Medium 4 |

Die auf diese Weise hergestellten Hauptkulturen wurden in einen Kuhner Inkubationsschüttler (Tabelle 13) überführt und inkubiert. Für die Probennahme über den Zeitraum der Kultivierung wurden die Schüttelkolben dem Inkubationsschüttler entnommen und gewogen, um Verdunstungseffekte zu ermitteln. Unter sterilen Bedingungen wurden Proben zur Bestimmung von Glucose, Xylose, ortho-Aminobenzoesäure und Biotrockenmasse entnommen.

### Ergebnisse

### _{D}-Xylose-Konzentration

**Tabelle 15: Übersicht über die _{D}-Xylosekonzentrationen über den zeitlichen Verlauf für je vier Replikate mit Medium 1 und Medium 2.**

| Bedingung (Gew.-% Zucker bezogen auf Gesamtzucker) | Medium 1 (100 Gew.-% _{D}-Glucose, 0 Gew.-% _{D}-Xylose) | | | | Medium 2 (64 Gew.-% _{D}-Glucose, 36 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | _{D}-Xylose (g/L) | _{D}-Xylose (g/L) | _{D}-Xylose (g/L) | _{D}-Xylose (g/L) | _{D}-Xylose (g/L) | _{D}-Xylose (g/L) | _{D}-Xylose (g/L) | _{D}-Xylose (g/L) |
| 0,00 | 0 | 0 | 0 | 0 | 7,53 | 7,28 | 7,36 | 7,46 |
| 19,00 | 0 | 0 | 0 | 0 | 7,20 | 7,05 | 7,09 | 7,24 |
| 26,00 | 0 | 0 | 0 | 0 | 7,22 | 7,15 | 7,18 | 7,38 |
| 42,83 | 0 | 0 | 0 | 0 | 5,37 | 5,20 | 5,25 | 5,43 |
| 50,00 | 0 | 0 | 0 | 0 | 4,19 | 4,06 | 4,09 | 4,21 |
| 66,50 | 0 | 0 | 0 | 0 | 2,41 | 2,22 | 2,26 | 2,51 |

### _{D}-Xylose-Konzentration

**Tabelle 16: Übersicht über die _{D}-Xylosekonzentrationen über den zeitlichen Verlauf für je vier Replikate mit Medium 3 und Medium 4.**

| Bedingung (Gew.-% Zucker bezogen auf Gesamtzucker) | Medium 3 (75 Gew.-% _{D}-Glucose, 25 Gew.-% _{D}-Xylose) | | | | Medium 4 (88 Gew.-% _{D}-Glucose, 12 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | _{D}-Xylose (g/L) | _{D}-Xylose (g/L) | _{D}-Xylose (g/L) | _{D}-Xylose (g/L) | _{D}-Xylose (g/L) | _{D}-Xylose (g/L) | _{D}-Xylose (g/L) | _{D}-Xylose (g/L) |
| 0,00 | 5,15 | 5,11 | 5,10 | 5,11 | 2,51 | 2,51 | 2,54 | 2,51 |
| 19,00 | 4,95 | 4,94 | 4,94 | 4,96 | 2,34 | 2,38 | 2,37 | 2,39 |
| 26,00 | 4,88 | 4,89 | 4,89 | 4,90 | 2,24 | 2,28 | 2,26 | 2,28 |
| 42,83 | 3,31 | 3,32 | 3,30 | 3,18 | 1,08 | 1,16 | 1,07 | 1,20 |
| 50,00 | 2,15 | 2,16 | 2,15 | 2,03 | 0,46 | 0,48 | 0,44 | 0,49 |
| 66,50 | 0,83 | 0,84 | 0,81 | 0,75 | 0,10 | 0,10 | 0,08 | 0,10 |

### _{D}-Glucose-Konzentration

**Tabelle 17: Übersicht über die _{D}-Glucosekonzentrationen über den zeitlichen Verlauf für je vier Replikate mit Medium 1 und Medium 2.**

| Bedingung (Gew.-% Zucker bezogen auf Gesamtzucker) | Medium 1 (100 Gew.-% _{D}-Glucose, 0 Gew.-% _{D}-Xylose) | | | | Medium 2 (64 Gew.-% _{D}-Glucose, 36 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | _{D}-Glucose (g/L) | _{D}-Glucose (g/L) | _{D}-Glucose (g/L) | _{D}-Glucose (g/L) | _{D}-Glucose (g/L) | _{D}-Glucose (g/L) | _{D}-Glucose (g/L) | _{D}-Glucose (g/L) |
| 0,00 | 21,46 | 21,46 | 21,46 | 21,46 | 13,19 | 13,19 | 13,19 | 13,19 |
| 19,00 | 16,08 | 16,51 | 16,23 | 16,62 | 9,18 | 8,90 | 9,03 | 9,37 |
| 26,00 | 12,38 | 12,56 | 12,40 | 12,89 | 6,21 | 5,95 | 6,15 | 6,44 |
| 42,83 | 0,00 | 0,00 | 0,00 | 0,00 | 0,00 | 0,00 | 0,00 | 0,00 |
| 50,00 | 0,00 | 0,00 | 0,00 | 0,00 | 0,00 | 0,00 | 0,00 | 0,00 |
| 66,50 | 0,00 | 0,00 | 0,00 | 0,00 | 0,00 | 0,00 | 0,00 | 0,00 |

### _{D}-Glucose-Konzentration

**Tabelle 18: Übersicht über die _{D}-Glucosekonzentrationen über den zeitlichen Verlauf für je vier Replikate mit Medium 3 und Medium 4.**

| Bedingung (Gew.-% Zucker bezogen auf Gesamtzucker) | Medium 3 (75 Gew.-% _{D}-Glucose, 25 Gew.-% _{D}-Xylose) | | | | Medium 4 (88 Gew.-% _{D}-Glucose, 12 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | _{D}-Glucose (g/L) | _{D}-Glucose (g/L) | _{D}-Glucose (g/L) | _{D}-Glucose (g/L) | _{D}-Glucose (g/L) | _{D}-Glucose (g/L) | _{D}-Glucose (g/L) | _{D}-Glucose (g/L) |
| 0,00 | 15,78 | 15,78 | 15,78 | 15,78 | 18,27 | 18,27 | 18,27 | 18,27 |
| 19,00 | 11,64 | 11,65 | 11,62 | 11,57 | 13,80 | 13,99 | 13,68 | 14,024 |
| 26,00 | 8,47 | 8,49 | 8,48 | 8,38 | 10,26 | 10,53 | 10,21 | 10,61937 |
| 42,83 | 0,00 | 0,00 | 0,00 | 0,00 | 0,00 | 0,00 | 0,00 | 0 |
| 50,00 | 0,00 | 0,00 | 0,00 | 0,00 | 0,00 | 0,00 | 0,00 | 0 |
| 66,50 | 0,00 | 0,00 | 0,00 | 0,00 | 0,00 | 0,00 | #NV | 0 |

Biotrockenmasse-Konzentration (BTM)

**Tabelle 19: Finale Biotrockenmassekonzentrationen in Doppelbestimmung für je vier Replikate mit Medium 1 und Medium 2.**

| Bedingung (Gew.-% Zucker bezogen auf Gesamtzucker) | Medium 1 (100 Gew.-% _{D}-Glucose, 0 Gew.-% _{D}-Xylose) | | | | Medium 2 (64 Gew.-% _{D}-Glucose, 36 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | BTM (g/L) | BTM (g/L) | BTM (g/L) | BTM (g/L) | BTM (g/L) | BTM (g/L) | BTM (g/L) | BTM (g/L) |
| 66,50 | 4,91 | 5,21 | 4,85 | 5,14 | 4,22 | 4,25 | 3,93 | 4,02 |
| 66,50 | 5,01 | 5,26 | 4,84 | 4,97 | 3,72 | 4,12 | 4,01 | 4,16 |

Biotrockenmasse-Konzentration (BTM)

**Tabelle 20: Finale Biotrockenmassekonzentrationen in Doppelbestimmung für je vier Replikate mit Medium 3 und Medium 4.**

| Bedingung (Gew.-% Zucker bezogen auf Gesamtzucker) | Medium 3 (75 Gew.-% _{D}-Glucose, 25 Gew.-% _{D}-Xylose) | | | | Medium 4 (88 Gew.-% _{D}-Glucose, 12 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | BTM (g/L) | BTM (g/L) | BTM (g/L) | BTM (g/L) | BTM (g/L) | BTM (g/L) | BTM (g/L) | BTM (g/L) |
| 66,50 | 4,61 | 4,39 | 4,46 | 4,48 | 4,76 | 4,4 | 4,7 | 4,7 |
| 66,50 | 4,49 | 4,34 | 4,41 | 4,56 | 4,67 | 4,62 | 4,81 | 4,88 |

### ortho-Aminobenzoesäure-Konzentration (oAb)

**Tabelle 21: Übersicht über die ortho-Aminobenzoesäure-Konzentration über den zeitlichen Verlauf für je vier Replikate mit Medium 1 und Medium 2.**

| Bedingung (Gew.-% Zucker bezogen auf Gesamtzucker) | Medium 1 (100 Gew.-% _{D}-Glucose, 0 Gew.-% _{D}-Xylose) | | | | Medium 2 (64 Gew.-% _{D}-Glucose, 36 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | oAb (g/L) | oAb (g/L) | oAb (g/L) | oAb (g/L) | oAb (g/L) | oAb (g/L) | oAb (g/L) | oAb (g/L) |
| 0,00 | 0,27 | 0,27 | 0,26 | 0,26 | 0,26 | 0,25 | 0,25 | 0,24 |
| 19,00 | 0,34 | 0,33 | 0,32 | 0,34 | 0,18 | 0,30 | 0,28 | 0,26 |
| 26,00 | 0,62 | 0,62 | 0,61 | 0,60 | 0,53 | 0,52 | 0,51 | 0,51 |
| 42,83 | 2,15 | 2,21 | 2,15 | 2,18 | 1,68 | 1,68 | 1,74 | 1,73 |
| 50,00 | 2,56 | 2,55 | 2,41 | 2,47 | 2,11 | 2,08 | 2,13 | 2,09 |
| 66,50 | 2,31 | 2,35 | 2,30 | 2,37 | 2,40 | 2,38 | 2,36 | 2,42 |

### ortho-Aminobenzoesäure-Konzentration (oAb)

**Tabelle 22: Übersicht über die ortho-Aminobenzoesäure-Konzentration über den zeitlichen Verlauf für je vier Replikate mit Medium 3 und Medium 4.**

| Bedingung (Gew.-% Zucker bezogen auf Gesamtzucker) | Medium 3 (75 Gew.-% _{D}-Glucose, 25 Gew.-% _{D}-Xylose) | | | | Medium 4 (88 Gew.-% _{D}-Glucose, 12 Gew.-% _{D}-Xylose) | | | |
|---|---|---|---|---|---|---|---|---|
| Replikat (-) | 1 | 2 | 3 | 4 | 1 | 2 | 3 | 4 |
| Kultivierungszeit (h) | oAb (g/L) | oAb (g/L) | oAb (g/L) | oAb (g/L) | oAb (g/L) | oAb (g/L) | oAb (g/L) | oAb (g/L) |
| 0,00 | 0,25 | 0,26 | 0,27 | 0,27 | 0,27 | 0,27 | 0,28 | 0,26 |
| 19,00 | 0,28 | 0,27 | 0,28 | 0,28 | 0,28 | 0,28 | 0,29 | 0,27 |
| 26,00 | 0,53 | 0,53 | 0,53 | 0,54 | 0,56 | 0,56 | 0,58 | 0,55 |
| 42,83 | 1,80 | 1,81 | 1,81 | 1,94 | 2,07 | 2,02 | 2,13 | 1,97 |
| 50,00 | 2,39 | 2,36 | 2,39 | 2,41 | 2,41 | 2,43 | 2,48 | 2,52 |
| 66,50 | 2,51 | 2,50 | 2,51 | 2,62 | 2,46 | 2,48 | 2,46 | 2,43 |

### Finale Produktausbeute

**Tabelle 23: Übersicht über die ortho-Aminobenzoesäureausbeuten nach 66,50 h für vier Replikate mit Medium 1 und Medium 2. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Bedingung (Gew.-% Zucker bezogen auf Gesamtzucker) | Medium 1 (100 Gew.-% _{D}-Glucose, 0 Gew.-% _{D}-Xylose) | | | | Medium 2 (64 Gew.-% _{D}-Glucose, 36 Gew.-% _{D}-Xylose) | | | |
|---|---|---|---|---|---|---|---|---|
| Replikat (-) | 1 | 2 | 3 | 4 | 1 | 2 | 3 | 4 |
| Ausbeute nach 66,50 h Kultivierung (g_{oAb}/g_{C-Quelle verbraucht}) | 0,0857 | 0,0881 | 0,0860 | 0,0900 | 0,1054 | 0,1044 | 0,1035 | 0,1090 |
| Ausbeute nach 66,50 h Kultivierung (g_{oAb}/g_{C-Quelle vorgelegt}) | 0,0819 | 0,0841 | 0,0822 | 0,0858 | 0,0890 | 0,0890 | 0,0880 | 0,0914 |
| Ausbeute nach 66,50 h Kultivierung (mol_{oAb}/mol_{C- Quelle verbraucht}) | 0,1126 | 0,1157 | 0,1130 | 0,1132 | 0,1312 | 0,1300 | 0,1288 | 0,1359 |
| Ausbeute nach 66,50 h Kultivierung (mol_{oAb}/mol_{C- Quelle vorbelegt}) | 0,1075 | 0,1105 | 0,1079 | 0,1128 | 0,1090 | 0,1091 | 0,1079 | 0,1120 |

### Finale Produktausbeute

**Tabelle 24: Übersicht über die ortho-Aminobenzoesäureausbeuten nach 66,50 h für vier Replikate mit Medium 3 und Medium 4. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Bedingung (Gew.-% Zucker bezogen auf Gesamtzucker) | Medium 3 (75 Gew.-% _{D}-Glucose, 25 Gew.-% _{D}-Xylose) | | | | Medium 4 (88 Gew.-% _{D}-Glucose, 12 Gew.-% _{D}-Xylose) | | | |
|---|---|---|---|---|---|---|---|---|
| Replikat (-) | 1 | 2 | 3 | 4 | 1 | 2 | 3 | 4 |
| Ausbeute nach 66,50 h Kultivierung (g_{oAb}/g_{C-Quelle verbraucht}) | 0,1020 | 0,0999 | 0,1017 | 0,1055 | 0,0949 | 0,0967 | 0,0943 | 0,0949 |
| Ausbeute nach 66,50 h Kultivierung (g_{oAb}/g_{C-Quelle vorgelegt}) | 0,0933 | 0,0927 | 0,0930 | 0,0968 | 0,0901 | 0,0917 | 0,0865 | 0,0900 |
| Ausbeute nach 66,50 h Kultivierung (mol_{oAb}/mol_{C-Quelle verbraucht}) | 0,1286 | 0,1253 | 0,1282 | 0,1329 | 0,1219 | 0,1242 | 0,1210 | 0,1219 |
| Ausbeute nach 66,50 h Kultivierung (mol_{oAb}/mol_{C-Quelle vorgelegt}) | 0,1168 | 0,1161 | 0,1165 | 0,1212 | 0,1155 | 0,1176 | 0,1147 | 0,1154 |

### Mittelwerte die finalen Produktausbeuten und Standardabweichungen

**Tabelle 25: Übersicht über die Mittelwerte und Standardabweichungen der ortho-Aminobenzoesäureausbeuten aus je vier Replikaten nach 66,50 h. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Medium Nr. (-) | Medium 1 | Medium 2 | Medium 3 | Medium 4 |
|---|---|---|---|---|
| Bedingung (Gew.-% Zucker bezogen auf Gesamtzucker) | 100 Gew.-% _{D}-Glucose, 0 Gew.-% _{D}-Xylose | 64 Gew.-% _{D}-Glucose, 36 Gew.-% _{D}-Xylose | 75 Gew.-% _{D}-Glucose, 25 Gew.-% _{D}-Xylose | 88 Gew.-% _{D}-Glucose, 12 Gew.-% _{D}-Xylose |
| Mittelwert der Ausbeute nach 66,50 h Kultivierung (g_{oAb}/g_{C-Quelle verbraucht}) | 0,087 ± 0,002 | 0,106 ± 0,002 | 0,102 ± 0,002 | 0,095 ± 0,001 |
| Mittelwert der Ausbeute nach 66,50 h Kultivierung (g_{oAb}/g_{C-Quelle vorgelegt}) | 0,083 ± 0,002 | 0,089 ± 0,001 | 0,094 ± 0,002 | 0,090 ± 0,001 |
| Mittelwert der Ausbeute nach 66,50 h Kultivierung (mol_{oAb}/mol_{C-Quelle verbraucht}) | 0,114 ± 0,001 | 0,131 ± 0,003 | 0,129 ± 0,003 | 0,122 ± 0,001 |
| Mittelwert der Ausbeute nach 66,50 h Kultivierung (mol_{oAb}/mol_{C-Quelle vorgelegt}) | 0,110 ± 0,002 | 0,110 ± 0,002 | 0,118 ± 0,002 | 0,116 ± 0,001 |

### Zusammenfassung

In der hier beschriebenen Schüttelkolbenkultivierung eines xyloseverstoffwechselnden ortho-Aminobenzoesäureproduzenten basierend auf *C*. *glutamicum* wurde der Einfluss verschiedener Mischungsverhältnisse von Glucose und Xylose auf die Ausbeute von ortho-Aminobenzoesäure untersucht.

Bis zur Beendigung der Kultivierungen wurde die Xylose in den meisten Fällen noch nicht vollständig verbraucht, weshalb bei der finalen Produktausbeute zwischen der "Substratausbeute" bezogen auf die Masse bzw. Stoffmenge des verbrauchten Substrats (g_{oAb} / g_{verbrauchte C-Quelle} bzw. mol_{oAb} / mol_{verbrauchte C-Quelle}) und der "Prozessausbeute" bezogen auf die Masse bzw. Stoffmenge des vorgelegten Substrats (g_{oAb} / g_{vorgelegte C-Quelle} bzw. mol_{oAb} / mol_{vorgelegte C-Quelle}) unterschieden wurde.

Es wurde festgestellt, dass die Substratausbeute bei Verwendung von Medium 2 mit 0,106 g_{oAb} / g_{verbrauchte C-Quelle} bzw. 0,129 mol_{oAb} / mol_{verbrauchte C-Quelle} am höchsten war. Die erreichte Biotrockenmassekonzentration nahm mit zunehmendem Xyloseanteil ab. Bei der Prozessausbeute wurde ein Maximum von 0,094 g_{oAb} / g_{vorgelegte C-Quelle} bzw. 0,118 mol_{oAb} / mol_{vorgelegte C-Quelle} mit Medium 3 erreicht, in dem die eingesetzte Energie- und Kohlenstoffquelle zu 76 Gew.-% aus Glukose und 24 Gew.-% Xylose zusammengesetzt war.

### Beispiel 2

Es wurden vergleichende Kultivierungen des oben beschriebenen Stamms zur Produktion von ortho-Aminobenzoesäure ausgehend von zwei verschiedenen Zuckern in acht verschiedenen Verhältnissen durchgeführt. Dabei wurden die Hauptkulturmedien so hergestellt, dass die beiden Zucker _{D}-Glucose und _{D}-Xylose in Mengen wie in Tabelle 26 bezogen auf den Gesamtzuckergehalt von 20 g/L vorlagen.

**Tabelle 26: Übersicht über die Zusammensetzungen der verschiedenen Zucker in den verwendeten Hauptkultivierungsmedien.**

| | Gew.-% Zucker (Anteil bezogen auf den Gesamtzuckergehalt) | |
|---|---|---|
| Bezeichnung Medium | _{D}-Glucose | _{D}-Xylose |
| Medium 1 | 100 | 0 |
| Medium 2 | 86 | 14 |
| Medium 3 | 76 | 24 |
| Medium 4 | 63 | 37 |
| Medium 5 | 50 | 50 |
| Medium 6 | 39 | 61 |
| Medium 7 | 27 | 73 |
| Medium 8 | 14 | 86 |

Für jede Bedingung bzw. jedes Zuckermengenverhältnis wurden je vier Replikate angelegt. Die Kultivierungsbedingungen sind nachfolgend dargestellt.

**Tabelle 27: Einstellungen am BioLector Pro für die Kultivierung der 48 Mikrotiterplatte.**

| Parameter | Einstellung |
|---|---|
| Temperatur (°C) | 30 |
| Schüttelfrequenz (rpm) | 1000 |
| Kultivierungszeit (h) | 70,79 |
| Luftfeuchtigkeit (%) | 85 |
| Zeit zwischen Messzyklen (min) | 5 |
| Sauerstoff in Kultivierungskammer (Gew.-%) | 21 |

**Tabelle 28: Layout der Kultivierungsplatte des Typs M2P-MTP-48-BOH2. An jeder Position wurde das gleiche Volumen von 1 mL der Hauptkultur eingesetzt.**

| | **1** | **2** | **3** | **4** | **5** | **6** | **7** | **8** |
|---|---|---|---|---|---|---|---|---|
| **A** | Medium 1 | Medium 1 | Medium 1 | Medium 1 | Medium 1 | Medium 1 | Medium 2 | Medium 2 |
| **B** | Medium 2 | Medium 2 | Medium 2 | Medium 2 | Medium 3 | Medium 3 | Medium 3 | Medium 3 |
| **C** | Medium 3 | Medium 3 | Medium 4 | Medium 4 | Medium 4 | Medium 4 | Medium 4 | Medium 4 |
| **D** | Medium 5 | Medium 5 | Medium 5 | Medium 5 | Medium 5 | Medium 5 | Medium 6 | Medium 6 |
| **E** | Medium 6 | Medium 6 | Medium 6 | Medium 6 | Medium 7 | Medium 7 | Medium 7 | Medium 7 |
| **F** | Medium 7 | Medium 7 | Medium 8 | Medium 8 | Medium 8 | Medium 8 | Medium 8 | Medium 8 |

### Verwendete Medien, deren Zusammensetzung und Herstellung

Wenn nicht anders beschrieben, werden alle Medien mit ddH₂O hergestellt und autoklaviert.

**Tabelle 29: Flüssige und feste Komplexmedien aus Brain-Heart-Infusion (BHI) zur Anzucht von Zellen.**

| **Medium** | **Zusammensetzung** | **Zugabe vor/nach Autoklav.** |
|---|---|---|
| **BHI-Broth** | 37 g/L BHI-Broth | Vor dem Autoklavieren |
| **BHI-Agarplatten** | 52 g/L BHI-Agar | Vor dem Autoklavieren |

**Tabelle 30: Flüssiges Minimalmedium mit Komplexbestandteilen für die Anzucht von Zellen in der Vorkultur. Die Mengen der Einwaage sind für 1 L fertiges CGXII Vorkulturmedium angegeben. Nachdem alle Reagenzien im Medium supplementiert wurden, werden die final angestrebten Konzentrationen im fertigen Medium erreicht.**

| **Medium** | **Zusammensetzung** | **Entspricht finaler Konzentration im fertigen Medium** | **Zugabe vor/nach Autoklav.** |
|---|---|---|---|
| **CGXII Vorkulturmedium** | 1 g KH₂PO₄ | 1 g/L | Ab hier alles vor dem Autoklavieren hinzu. |
| | 1 g K₂HPO₄ | 1 g/L | |
| | 10 g (NH₄)₂SO₄ | 10 g/L | |
| | 5 g Urea | 5 g/L | |
| | 62 g MOPS | 62 g/L | |
| | 5 g Hefeextrakt | 5 g/L | |
| | 1 mL CaCl₂-Stammlösung | 1 mL/L | |
| **In 800 mL ddH₂O lösen, pH mit KOH-Plättchen einstellen auf pH=7,0 - 7,5, mit ddH₂O auffüllen auf 940 mL und autoklavieren.** | | | |
| | 1,25 mL MgSO₄-Stammlösung | 1,25 mL/L | Ab hier alles nach dem Autoklavieren hinzu: |
| | 1 mL Spurenelement-Stammlösung | 1 mL/L | |
| | 1 mL Biotin-Stammlösung | 1 mL/L | |
| | 33 mL _{D}-Glucose- ODER | 20 g/L _{D}-Glucose- ODER | |
| | 33 mL _{D}-Glucose und 16,5 mL _{D}-Xylose-Stammlösung | 20 g/L _{D}-Glucose und 10 g/L _{D}-Xylose | |
| | 23,75 mL Wasser ODER | - | |
| | 7,25 mL Wasser | | |

**Tabelle 31: Flüssiges Minimalmedium für die Hauptkultur. Die Mengen der Einwaage sind für 1 L fertiges CGXII Hauptkulturmedium angegeben. Nachdem alle Reagenzien im Medium supplementiert wurden, werden die final angestrebten Konzentrationen im fertigen Medium erreicht.**

| **Medium** | **Zusammensetzung** | **Entspricht finaler Konzentration im fertigen Medium** | **Zugabe vor/nach Autoklav.** |
|---|---|---|---|
| **CGXII Hauptkulturmedium** | 1 g KH₂PO₄ | 1 g/L | Ab hier alles vor dem Autoklavieren hinzu: |
| | 1 g K₂HPO₄ | 1 g/L | |
| | 10 g (NH₄)₂SO₄ | 10 g/L | |
| | 62 g MOPS | 62 g/L | |
| | 1 mL CaCl₂-Stammlösung | 1 mL/L | |
| **In 800 mL ddH₂O lösen, pH mit KOH-Plättchen einstellen auf pH=7,0 - 7,5, mit ddH₂O auffüllen auf 940 mL und autoklavieren.** | | | |
| | 1,25 mL MgSO₄-Stammlösung | 1,25 mL/L | Ab hier alles nach dem Autoklavieren: |
| | 1 mL Spurenelement-Stammlösung | 1 mL/L | |
| | 1 mL Biotin-Stammlösung | 1 mL/L | |
| | 33 mL _{D}-Glucose- ODER | 20 g/L _{D}-Glucose- ODER | |
| | 33 mLD-Xylose-Stammlösung | 20 g/L _{D}-Xylose | |
| | 23,75 mL Wasser | - | |

**Tabelle 32: Übersicht zur Herstellung der 2 g/L Biotin-Stammlösung. Die Lösung wird nicht autoklaviert, sondern steril filtriert (0,2 µm). Die Lösung kann für 1 Monat bei 4 °C aufbewahrt werden.**

| **Medium** | **Zusammensetzung** | **Notiz** |
|---|---|---|
| **Biotin-Stammlösung** | 2 g/L Biotin | Lösung wird steril filtriert. |
| | | Während des Lösens wird unter Rühren der pH-Wert gemessen und dieser mit 1 M KOH auf pH=7,2 eingestellt. |

**Tabelle 33: Übersicht zur Herstellung der 600 g/L _{D}-Glucose-Stammlösung.**

| **Medium** | **Zusammensetzung** | **Notiz** |
|---|---|---|
| **_{D}-Glucose-Stammlösung** | 660 g _{D}-Glucose-Monohgydrat | Substanz wird in 554 g heißem ddH₂O gelöst. Gesamtgewicht von 1 L der fertigen Lösung: 1214 g. Zum vollständigen Lösen vor dem Autoklavieren kurz Aufkochen. |

**Tabelle 34: Übersicht zur Herstellung der 600 g/L _{D}-Xylose-Stammlösung.**

| **Medium** | **Zusammensetzung** | **Notiz** |
|---|---|---|
| **_{D}-Xylose-Stammlösung** | 600 g _{D}-Xylose | Substanz wird in 595 g heißem ddH₂O gelöst. Gesamtgewicht von 1 L der fertigen Lösung: 1195 g. Zum vollständigen Lösen vor dem Autoklavieren kurz Aufkochen. |

**Tabelle 35: Übersicht zur Herstellung der 10 g/L CaCl₂-Stammlösung.**

| **Medium** | **Zusammensetzung** | **Notiz** |
|---|---|---|
| **CaCl₂-Stammlösung** | 10 g/L CaCl₂ × 2 H₂O | Lösung muss nicht autoklaviert werden, da sie vor dem Autoklaviervorgang der CGXII-Medien supplementiert wird. |

**Tabelle 36: Übersicht zur Herstellung der 200 g/L MgSO₄-Stammlösung.**

| **Medium** | **Zusammensetzung** | **Notiz** |
|---|---|---|
| **MgSO₄-Stammlösung** | 200 g/L MgSO₄ × 7 H₂O | Lösung wird steril filtriert. |

**Tabelle 37: Übersicht zur Herstellung der 1000x Spurenelement-Lösung. Die Lösung wird nicht autoklaviert, sondern steril filtriert (0,2 µm). Diese Lösung ist 6 Monate bei 4 °C haltbar. Empfehlenswert aufgrund der geringen Einwaagen ist die Herstellung eines Ansatzes von 1 L.**

| **Medium** | **Zusammensetzung** | **Notiz** |
|---|---|---|
| **Spurenelement-Lösung** | 10 g/L MnSO₄ × H₂O | Lösung wird steril filtriert. |
| | 10 g/L FeSO₄ × 7 H₂O | |
| | 1 g/L ZnSO₄ × 7 H₂O | |
| | 0,2 g/L CuSO₄ × 5 H₂O | |
| | 0,02 g/L NiCl₂ × 6 H₂O | |
| In ddH₂O lösen und pH mit HCl einstellen auf pH=1. | | |

**Tabelle 38: Übersicht zur Herstellung von 1x Phosphatpuffer (PBS). Verwendet wurde der 10x PBS (Artikelnummer BP399-1) der Firma Fisher Scientific GmbH.**

| **Medium** | **Zusammensetzung** | **Notiz** |
|---|---|---|
| **1x Phosphatpuffer** | 10x PBS | 1:10 verdünnen mit ddH₂O autoklavieren. |

### Verwendete Geräte

**Tabelle 39: Übersicht über die in dieser Studie untersuchten Parameter sowie die Geräte und Verfahren zu deren Untersuchung.**

| Parameter | Gerät | Beschreibung |
|---|---|---|
| Glucose-Konzentration | Cedex Bio Analyzer, Roche S/N 35163 | Assay Glucose Cedex Bio mit der Artikelnummer RD-06343732001 nach Herstelleranleitung verwendet |
| NH₃-Konzentration | Cedex Bio Analyzer, Roche S/N 35163 | Assay NH3 Cedex Bio mit der Artikelnummer RD-06343775001 nach Herstelleranleitung verwendet. |
| ortho-Aminobenzoesäure-Konzentration | HPLC, Agilent G7104C 1260 Flexible Pump S/N DEAGZ01520 G7167A 1260 Multisampler S/N DEAGY01567 G7116A 1260 Multicolumn Thermostat (MCT) S/N DEAEM05811 G7117C 1260 DAD HS S/N DEAEK06638 G7162A 1260 RID S/N DEAC904783 | Zur Trennung und Quantifizierung der ortho-Aminobenzoesäure im sterilfiltrierten Überstand der Proben wurden von der Firma Agilent eine Eclipse Plus C18 Säule (4,6 × 150 mm, 5 µm, S/N USUXB19935) und eine Zorbax Eclipse Plus C18 Vorsäulenkartusche (4,6 × 12 mm, 5 µm, 5 mm) verwendet. Die Detektion der ortho-Aminobenzoesäure erfolgte über den Diodenarray-Detektor (DAD). |
| _{D}-Xylose-Konzentration | HPLC, ROA HPLC, Agilent G7104C 1260 Flexible Pump S/N DEAGZ01520 G7167A 1260 Multisampler S/N DEAGY01567 G7116A 1260 Multicolumn Thermostat (MCT) S/N DEAEM05811 G7117C 1260 DAD HS S/N DEAEK06638 G7162A 1260 RID S/N DEAC904783 | Zur Trennung und Quantifizierung der _{D}-Xylose im sterilfiltrierten Überstand der Proben wurden von der Firma Phenomenex eine Rezex ROA-Organic Acid H+ 8 Säule (300 × 7,8 mm, S/N H20-094694) und eine SecurityGuard Cartridge Carbo-H Vorsäulenkartusche (4 × 3,0 mm) verwendet. Die Detektion der _{D}-Xylose erfolgte über den Brechungsindexdetektor (RID). |
| pH-Wert | pH-Meter SevenCompact S210, Mettler Toledo S/N B921689454 pH-Elektrode InLab Expert Pro-ISM, Mettler Toledo S/N 30014096 | |
| Gewicht Biotrockenmasse | SARTORIUS CUBIS^{®} Semimikrowaage 225S, Sartorius S/N 38101395 | |

**Tabelle 40: Übersicht über die verwendeten Produkte der Firma Beckman Coulter GmbH für die Kultivierung der Hauptkulturen.**

| Produkt | Beschreibung | Eigenschaften |
|---|---|---|
| G-BLMF100 | BioLector^{®} Pro system | Integrierte Mikrofluidik zur aktiven pH-Regelung und Substratzufütterung in der Mikrotiterplatte, 6 Filter für die folgenden Parameter: Biomasse, pH, DO (dissolved oxygen), Riboflavin (Ex 436 nm / Em 540 nm), LG1 (pH) and RF (DO); Die Inkubationskammer ist bestückt mit Sensoren für Feuchte und Temperatur; Befeuchtungsfunktion der Inkubationskammer (>75Gew.-%) |
| E-O2-100 | O2 Anreicherungsmodul Rev10.3 | Modul zur Anreicherung des Sauerstoffs (bis 35Gew.-%). inkl. O2-Sensor und Druckminderer |
| E-OP-498 | Konfigurierbares LED-Modul BL II und Pro | Konfigurierbares Filter-Modul für BioLector^{®} II und Pro: Die Wellenlängen für Emission und Excitation können zwischen 365 und 800 nm liegen (Bandpass-Filter: 10 nm). |
| M2P-MTP-48-BOH2 | FlowerPlate MTP, pH/DO type 2 (LG1/RF) | 48 well FlowerPlate Mikrotiterplatte, transparenter Boden, pH and DO Optoden Typ 2 (LG1/RF). |
| M2P-F-GPR48-10 | Sealing foil, Gas-permeable, Reduced evaporation | Selbstklebende, Gas-permeable Abdichtungsfolie, dient als Sterilbarriere und reduziert die Evaporation |

### Durchführung

Von Glycerinüberdauerungsformen der für die Kultivierung verwendeten mikrobiellen Kulturen wurde zur Generierung einer Starterkultur Zellmasse entnommen und diese zum Beimpfen einer BHI-Agarplatte verwendet. Die BHI-Agarplatte wurde bei 30 °C für 48 h inkubiert.

Die auf diese Weise angelegte Agarplattenkultur wurde zum Beimpfen einer BHI-Flüssigkultur verwendet. Dazu wurde der BHI-Agarplattenkultur etwas Zellmasse entnommen und 4 mL BHI-Flüssigmedium in einem Rundbodenröhrchen beimpft. Die Flüssigkultur wurde etwa 7,5 h bei 30 °C und 200 rpm inkubiert (Vorkultur I).

Für die zweite Vorkultur wurden zwei Medien mit verschiedenen Kohlenstoffquellen verwendet. Dazu wurde CGXII Vorkulturmedium mit entweder 20 g/L _{D}-Glucose oder mit 20 g/L _{D}-Glucose und zusätzlich 10 g/L _{D}-Xylose hergestellt und je 50 mL in einen 1 L-Erlenmeyerkolben überführt. Zu beiden Ansätzen wurden je 2 mL der BHI-Flüssigvorkultur gegeben und diese beiden Schüttelkolbenvorkulturen bei 200 rpm und 30 °C für 17 h inkubiert.

Nach erfolgter Inkubation der zweiten Vorkultur im Schüttelkolben wurde die optische Dichte der Kulturen, die entweder nur Glucose oder eine Mischung von Glucose und Xylose als Kohlenstoffquelle zur Verfügung hatten, gemessen. Abhängig von der erreichten Zelldichte wurde ein Anteil dieser zweiten Vorkulturen entnommen, die Zellen durch Zentrifugation pelletiert, in sterilem Phosphatpuffer (einfach-konzentriert) gewaschen und in entsprechendem Hauptkulturmedium (Medien 1-8) resuspendiert.

**Tabelle 41: Messung der optischen Dichte (OD₆₀₀) nach erfolgter Inkubation der zweiten Vorkultur im Schüttelkolben. Anhand der gemessenen optischen Dichte wurde berechnet, wie viel Volumen der zweiten Vorkultur nötig ist, um 10 mL Hauptkultur mit einer initialen OD₆₀₀ von 1 zu beimpfen. Da xylosehaltige Medien mit sieben unterschiedlichen Xylose-Konzentrationen getestet werden sollten, wurden von der Vorkultur mit 20 g/L D-Glucose und 10 g/L D-Xylose sieben Aliquots für die Inokulation entnommen.**

| | OD₆₀₀(-) | Benötigtes Volumen |
|---|---|---|
| 20 g/L _{D}-Glucose | 23,24 | 1 × 430 µL für Medium 1 |
| 20 g/L _{D}-Glucose und 10 g/L _{D}-Xylose | 18,17 | 7 × 550 für Medium 2-8 |

Die Vorkultur mit Glucose als einziger Kohlenstoff- und Energiequelle wurde für die Herstellung der Hauptkultur mit Medium 1 verwendet. Mittels der Vorkultur mit Glucose und Xylose wurden die Hauptkulturen mit den Medien 2-8 beimpft. Von der auf diese Weise hergestellten Hauptkulturen wurden jeweils 6 × 1 mL (6 Replikate pro Zuckermengenverhältnis) in verschiedene Positionen einer Kultivierungsplatte pipettiert, die Kultivierungsplatte mit einer Folie (**Fehler! Verweisquelle konnte nicht gefunden werden.)** versiegelt und in den BioLector pro überführt.

### Ergebnisse

### _{D}-Xylose-Konzentration

**Tabelle 42: Übersicht über die _{D}-Xylosekonzentrationen für die sechs Replikate mit Medium 1 und Medium 2 zu Beginn der Kultivierung und nach 70,79 h.**

| Bedingung (Gew.-% Zucker bezogen auf Gesamtzucker) | Medium 1 (100 Gew.-% _{D}-Glucose, 0 Gew.-% _{D}-Xylose) | | | | | | Medium 2 (86 Gew.-% _{D}-Glucose, 14 Gew.-% _{D}-Xylose) | | | | | |
|---|---|---|---|---|---|---|---|---|---|---|---|---|
| Position in MTP | A1 | A2 | A3 | A4 | A5 | A6 | A7 | A8 | B1 | B2 | B3 | B4 |
| _{D}-Xylose nach 0,00 h Kultivierung (g/L) | 0 | 0 | 0 | 0 | 0 | 0 | 2,983 | 2,983 | 2,983 | 2,983 | 2,983 | 2,983 |
| _{D}-Xylose nach 70,79 h Kultivierung (g/L) | 0 | 0 | 0 | 0 | 0 | 0 | 0,041 | 0,044 | 0,051 | 0,049 | 0,044 | 0,200 |

### _{D}-Xylose-Konzentration

**Tabelle 43: Übersicht über die _{D}-Xylosekonzentrationen für die sechs Replikate mit Medium 3 und Medium 4 zu Beginn der Kultivierung und nach 70,79 h.**

| Bedingung (Gew.-% Zucker bezogen auf Gesamtzucker) | Medium 3 (76 Gew.-% _{D}-Glucose, 24 Gew.-% _{D}-Xylose) | | | | | | Medium 4 (63 Gew.-% _{D}-Glucose, 37 Gew.-% _{D}-Xylose) | | | | | |
|---|---|---|---|---|---|---|---|---|---|---|---|---|
| Position in MTP | B5 | B6 | B7 | B8 | C1 | C2 | C3 | C4 | C5 | C6 | C7 | C8 |
| _{D}-Xylose nach 0,00 h Kultivierung (g/L) | 4,884 | 4,884 | 4,884 | 4,884 | 4,884 | 4,884 | 7,567 | 7,567 | 7,567 | 7,567 | 7,567 | 7,567 |
| _{D}-Xylose nach 70,79 h Kultivierung (g/L) | 0,398 | 0,567 | 0,549 | 0,522 | 0,410 | 0,740 | 1,809 | 1,694 | 2,164 | 2,160 | 2,086 | 2,440 |

### _{D}-Xylose-Konzentration

**Tabelle 44: Übersicht über die _{D}-Xylosekonzentrationen für die sechs Replikate mit Medium 1 und Medium 2 zu Beginn der Kultivierung und nach 70,79 h.**

| Bedingung (Gew.-% Zucker bezogen auf Gesamtzucker) | Medium 5 (50 Gew.-% _{D}-Glucose, 50 Gew.-% _{D}-Xylose) | | | | | | Medium 6 (39 Gew.-% _{D}-Glucose, 61 Gew.-% _{D}-Xylose) | | | | | |
|---|---|---|---|---|---|---|---|---|---|---|---|---|
| Position in MTP | D1 | D2 | D3 | D4 | D5 | D6 | D7 | D8 | E1 | E2 | E3 | E4 |
| _{D}-Xylose nach 0,00 h Kultivierung (g/L) | 10,39 | 10,39 | 10,39 | 10,39 | 10,39 | 10,39 | 12,29 | 12,29 | 12,29 | 12,29 | 12,29 | 12,29 |
| _{D}-Xylose nach 70,79 h Kultivierung (g/L) | 4,46 | 4,53 | 4,45 | 4,30 | 4,56 | 4,68 | 7,23 | 7,34 | 7,3 | 7,41 | 7,52 | 7,85 |

### _{D}-Xylose-Konzentration

**Tabelle 45: Übersicht über die _{D}-Xylosekonzentrationen für die sechs Replikate mit Medium 7 und Medium 8 zu Beginn der Kultivierung und nach 70,79 h.**

| Bedingung (Gew.-% Zucker bezogen auf Gesamtzucker) | Medium 7 (27 Gew.-% _{D}-Glucose, 73 Gew.-% _{D}-Xylose) | | | | | | Medium 8 (14 Gew.-% _{D}-Glucose, 86 Gew.-% _{D}-Xylose) | | | | | |
|---|---|---|---|---|---|---|---|---|---|---|---|---|
| Position in MTP | E5 | E6 | E7 | E8 | F1 | F2 | F3 | F4 | F5 | F6 | F7 | F8 |
| _{D}-Xylose nach 0,00 h Kultivierung (g/L) | 14,42 | 14,42 | 14,42 | 14,42 | 14,42 | 14,42 | 16,76 | 16,76 | 16,76 | 16,76 | 16,76 | 16,76 |
| _{D}-Xylose nach 70,79 h Kultivierung (g/L) | 10,79 | 10,66 | 10,68 | 10,77 | 10,73 | 10,19 | 14,33 | 14,73 | 14,51 | 14,65 | 14,68 | 15,47 |

### _{D}-Glucose-Konzentration

**Tabelle 46: Übersicht über die _{D}-Glucosekonzentrationen für die sechs Replikate mit Medium 1 und Medium 2 zu Beginn der Kultivierung und nach 70,79 h.**

| Bedingung (Gew.-% Zucker bezogen auf Gesamtzucker) | Medium 1 (100 Gew.-% _{D}-Glucose, 0 Gew.-% _{D}-Xylose) | | | | | | Medium 2 (86 Gew.-% _{D}-Glucose, 14 Gew.-% _{D}-Xylose) | | | | | |
|---|---|---|---|---|---|---|---|---|---|---|---|---|
| Position in MTP | A1 | A2 | A3 | A4 | A5 | A6 | A7 | A8 | B1 | B2 | B3 | B4 |
| _{D}-Glucose nach 0,00 h Kultivierung (g/L) | 21,05 | 21,05 | 21,05 | 21,05 | 21,05 | 21,05 | 18,31 | 18,31 | 18,31 | 18,31 | 18,31 | 18,31 |
| _{D}-Glucose nach 70,79 h Kultivierung (g/L) | 0 | 0 | 0 | 0 | 0 | 0 | 0 | 0 | 0 | 0 | 0 | 0 |

### _{D}-Glucose-Konzentration

**Tabelle 47: Übersicht über die _{D}-Glucosekonzentrationen für die sechs Replikate mit Medium 3 und Medium 4 zu Beginn der Kultivierung und nach 70,79 h.**

| Bedingung (Gew.-% Zucker bezogen auf Gesamtzucker) | Medium 3 (76 Gew.-% _{D}-Glucose, 24 Gew.-% _{D}-Xylose) | | | | | | Medium 4 (63 Gew.-% _{D}-Glucose, 37 Gew.-% _{D}-Xylose) | | | | | |
|---|---|---|---|---|---|---|---|---|---|---|---|---|
| Position in MTP | B5 | B6 | B7 | B8 | C1 | C2 | C3 | C4 | C5 | C6 | C7 | C8 |
| _{D}-Glucose nach 0,00 h Kultivierung (g/L) | 15,74 | 15,74 | 15,74 | 15,74 | 15,74 | 15,74 | 13,06 | 13,06 | 13,06 | 13,06 | 13,06 | 13,06 |
| _{D}-Glucose nach 70,79 h Kultivierung (g/L) | 0 | 0 | 0 | 0 | 0 | 0 | 0 | 0 | 0 | 0 | 0 | 0 |

### _{D}-Glucose-Konzentration

**Tabelle 48: Übersicht über die _{D}-Glucosekonzentrationen für die sechs Replikate mit Medium 5 und Medium 6 zu Beginn der Kultivierung und nach 70,79 h.**

| Bedingung (Gew.-% Zucker bezogen auf Gesamtzucker) | Medium 5 (50 Gew.-% _{D}-Glucose, 50 Gew.-% _{D}-Xylose) | | | | | | Medium 6 (39 Gew.-% _{D}-Glucose, 61 Gew.-% _{D}-Xylose) | | | | | |
|---|---|---|---|---|---|---|---|---|---|---|---|---|
| Position in MTP | D1 | D2 | D3 | D4 | D5 | D6 | D7 | D8 | E1 | E2 | E3 | E4 |
| _{D}-Glucose nach 0,00 h Kultivierung (g/L) | 10,46 | 10,46 | 10,46 | 10,46 | 10,46 | 10,46 | 7,81 | 7,81 | 7,81 | 7,81 | 7,81 | 7,81 |
| _{D}-Glucose nach 70,79 h Kultivierung (g/L) | 0 | 0 | 0 | 0 | 0 | 0 | 0 | 0 | 0 | 0 | 0 | 0 |

### _{D}-Glucose-Konzentration

**Tabelle 49: Übersicht über die _{D}-Glucosekonzentrationen für die sechs Replikate mit Medium 7 und Medium 8 zu Beginn der Kultivierung und nach 70,79 h.**

| Bedingung (Gew.-% Zucker bezogen auf Gesamtzucker) | Medium 7 (27 Gew.-% _{D}-Glucose, 73 Gew.-% _{D}-Xylose) | | | | | | Medium 8 (14 Gew.-% _{D}-Glucose, 86 Gew.-% _{D}-Xylose) | | | | | |
|---|---|---|---|---|---|---|---|---|---|---|---|---|
| Position in MTP | E5 | E6 | E7 | E8 | F1 | F2 | F3 | F4 | F5 | F6 | F7 | F8 |
| _{D}-Glucose nach 0,00 h Kultivierung (g/L) | 5,25 | 5,25 | 5,25 | 5,25 | 5,25 | 5,25 | 2,63 | 2,63 | 2,63 | 2,63 | 2,63 | 2,63 |
| _{D}-Glucose nach 70,79 h Kultivierung (g/L) | 0 | 0 | 0 | 0 | 0 | 0 | 0 | 0 | 0 | 0 | 0 | 0 |

### Biotrockenmasse-Konzentration (BTM)

**Tabelle 50: Übersicht über die Biotrockenmassekonzentrationen für die sechs Replikate mit Medium 1 und Medium 2 nach 70,79 h.**

| Bedingung (Gew.-% Zucker bezogen auf Gesamtzucker) | Medium 1 (100 Gew.-% _{D}-Glucose, 0 Gew.-% _{D}-Xylose) | | | | | | Medium 2 (86 Gew.-% _{D}-Glucose, 14 _{D}-Xylose) | | | | | |
|---|---|---|---|---|---|---|---|---|---|---|---|---|
| Position in MTP | A1 | A2 | A3 | A4 | A5 | A6 | A7 | A8 | B1 | B2 | B3 | B4 |
| BTM nach 70,79 h Kultivierung (g/L) | 6,71 | 5,72 | 5,63 | 5,69 | 6,12 | 5,96 | 5,05 | 5,09 | 4,56 | 5,20 | 5,49 | 4,76 |

### Biotrockenmasse-Konzentration (BTM)

**Tabelle 51: Übersicht über die Biotrockenmassekonzentrationen für die sechs Replikate mit Medium 3 und Medium 4 nach 70,79 h.**

| Bedingung (Gew.-% Zucker bezogen auf Gesamtzucker) | Medium 3 (76 Gew.-% _{D}-Glucose, 24 Gew.-% _{D}-Xylose) | | | | | | Medium 4 (63 Gew.-% _{D}-Glucose, 37 Gew.-% _{D}-Xylose) | | | | | |
|---|---|---|---|---|---|---|---|---|---|---|---|---|
| Position in MTP | B5 | B6 | B7 | B8 | C1 | C2 | C3 | C4 | C5 | C6 | C7 | C8 |
| BTM nach 70,79 h Kultivierung (g/L) | 5,37 | 4,95 | 5,36 | 4,55 | 5,37 | 6,17 | 5,11 | 4,75 | 4,64 | 4,79 | 5,56 | 4,11 |

### Biotrockenmasse-Konzentration (BTM)

**Tabelle 52: Übersicht über die Biotrockenmassekonzentrationen für die sechs Replikate mit Medium 5 und Medium 6 nach 70,79 h.**

| Bedingung (Gew.-% Zucker bezogen auf Gesamtzucker) | Medium 5 (50 Gew.-% _{D}-Glucose, 50 Gew.-% _{D}-Xylose) | | | | | | Medium 6 (39 Gew.-% _{D}-Glucose, 61 Gew.-% _{D}-Xylose) | | | | | |
|---|---|---|---|---|---|---|---|---|---|---|---|---|
| Position in MTP | D1 | D2 | D3 | D4 | D5 | D6 | D7 | D8 | E1 | E2 | E3 | E4 |
| BTM nach 70,79 h Kultivierung (g/L) | 5,33 | 4,43 | 4,01 | 4,27 | 4,19 | 4,73 | 3,88 | 2,09 | 3,52 | 2,63 | 3,57 | 3,19 |

### Biotrockenmasse-Konzentration (BTM)

**Tabelle 53: Übersicht über die Biotrockenmassekonzentrationen für die sechs Replikate mit Medium 7 und Medium 8 nach 70,79 h.**

| Bedingung (Gew.-% Zucker bezogen auf Gesamtzucker) | Medium 7 (27 Gew.-% _{D}-Glucose, 73 Gew.-% _{D}-Xylose) | | | | | | Medium 8 (14 Gew.-% _{D}-Glucose, 86 Gew.-% _{D}-Xylose) | | | | | |
|---|---|---|---|---|---|---|---|---|---|---|---|---|
| Position in MTP | E5 | E6 | E7 | E8 | F1 | F2 | F3 | F4 | F5 | F6 | F7 | F8 |
| BTM nach 0,00 h Kultivierung (g/L) | #NV | #NV | #NV | #NV | #NV | #NV | #NV | #NV | #NV | #NV | #NV | #NV |
| BTM nach 70,79 h Kultivierung (g/L) | 2,68 | 2,37 | 2,37 | 2,29 | 2,84 | 3,68 | #NV | 1,49 | 2,63 | 1,17 | 1,56 | 1,49 |

### ortho-Aminobenzoesäure-Konzentration (oAB)

**Tabelle 54: Übersicht über die ortho-Aminobenzoesäurekonzentration für die sechs Replikate mit Medium 1 und Medium 2 nach 70,79 h.**

| Bedingung (Gew.-% Zucker bezogen auf Gesamtzucker) | Medium 1 (100 Gew.-% _{D}-Glucose, 0 Gew.-% _{D}-Xylose) | | | | | | Medium 2 (86 Gew.-% _{D}-Glucose, 14 Gew.-% _{D}-Xylose) | | | | | |
|---|---|---|---|---|---|---|---|---|---|---|---|---|
| Position in MTP | A1 | A2 | A3 | A4 | A5 | A6 | A7 | A8 | B1 | B2 | B3 | B4 |
| oAB nach 70,79 h Kultivierung (g/L) | 2,258 | 2,295 | 2,341 | 2,245 | 2,345 | 2,322 | 2,464 | 2,450 | 2,427 | 2,409 | 2,450 | 2,432 |

### ortho-Aminobenzoesäure-Konzentration (oAB)

**Tabelle 55: Übersicht über die ortho-Aminobenzoesäurekonzentration für die sechs Replikate mit Medium 3 und Medium 4 nach 70,79 h.**

| Bedingung (Gew.-% Zucker bezogen auf Gesamtzucker) | Medium 3 (76 Gew.-% _{D}-Glucose, 24 Gew.-% _{D}-Xylose) | | | | | | Medium 4 (63 Gew.-% _{D}-Glucose, 37 Gew.-% _{D}-Xylose) | | | | | |
|---|---|---|---|---|---|---|---|---|---|---|---|---|
| Position in MTP | B5 | B6 | B7 | B8 | C1 | C2 | C3 | C4 | C5 | C6 | C7 | C8 |
| oAB nach 70,79 h Kultivierung (g/L) | 2,425 | 2,469 | 2,430 | 2,448 | 2,441 | 2,400 | 2,341 | 2,336 | 2,343 | 2,315 | 2,357 | 2,345 |

### ortho-Aminobenzoesäure-Konzentration (oAB)

**Tabelle 56: Übersicht über die ortho-Aminobenzoesäurekonzentration für die sechs Replikate mit Medium 5 und Medium 6 nach 70,79 h.**

| Bedingung (Gew.-% Zucker bezogen auf Gesamtzuck er) | Medium 5 (50 Gew.-% _{D}-Glucose, 50 Gew.-% _{D}-Xylose) | | | | | | Medium 6 (39 Gew.-% _{D}-Glucose, 61 Gew.-% _{D}-Xylose) | | | | | |
|---|---|---|---|---|---|---|---|---|---|---|---|---|
| Position in MTP | D1 | D2 | D3 | D4 | D5 | D6 | D7 | D8 | E1 | E2 | E3 | E4 |
| oAB nach 70,79 h Kultivierung (g/L) | 2,11 9 | 2,10 3 | 2,09 8 | 2,08 5 | 2,08 2 | 2,13 7 | 1,78 3 | 1,75 8 | 1,77 8 | 1,75 8 | 1,71 7 | 1,71 9 |

### ortho-Aminobenzoesäure-Konzentration (oAB)

**Tabelle 57: Übersicht über die ortho-Aminobenzoesäurekonzentration für die sechs Replikate mit Medium 7 und Medium 8 nach 70,79 h.**

| Bedingung (Gew.-% Zucker bezogen auf Gesamtzucker) | Medium 7 (27 Gew.-% _{D}-Glucose, 73 Gew.-% _{D}-Xylose) | | | | | | Medium 8 (14 Gew.-% _{D}-Glucose, 86 Gew.-% _{D}-Xylose) | | | | | |
|---|---|---|---|---|---|---|---|---|---|---|---|---|
| Position in MTP | E5 | E6 | E7 | E8 | F1 | F2 | F3 | F4 | F5 | F6 | F7 | F8 |
| oAB nach 70,79 h Kultivierung (g/L) | 1,305 | 1,323 | 1,327 | 1,320 | 1,375 | 1,397 | 0,876 | 0,790 | 0,821 | 0,801 | 0,771 | 0,773 |

### Finale Produktausbeute

**Tabelle 58: Übersicht über die ortho-Aminobenzoesäureausbeuten nach 70,79 h für sechs Replikate mit Medium 1 und Medium 2. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Bedingun g (Gew.-% Zucker bezogen auf Gesamtzu cker) | Medium 1 (100 Gew.-% _{D}-Glucose, 0 Gew.-% _{D}-Xylose) | | | | | | Medium 2 (86 Gew.-% _{D}-Glucose, 14 Gew.-% _{D}-Xylose) | | | | | |
|---|---|---|---|---|---|---|---|---|---|---|---|---|
| Position in MTP | A1 | A2 | A3 | A4 | A5 | A6 | A7 | A8 | B1 | B2 | B3 | B4 |
| Ausbeute nach 70,79 h Kultivieru ng (g_{oAb}/g_{C-Quelle verbraucht}) | 0,107 | 0,109 | 0,111 | 0,107 | 0,111 | 0,110 | 0,116 | 0,115 | 0,114 | 0,113 | 0,115 | 0,115 |
| Ausbeute nach 70,79 h Kultivieru ng (g_{oAb}/g_{C-Quelle vorgelegt}) | 0,107 | 0,109 | 0,111 | 0,107 | 0,111 | 0,110 | 0,116 | 0,115 | 0,114 | 0,113 | 0,115 | 0,114 |
| Ausbeute nach 70,79 h Kultivieru ng (mol_{oAb}/m ol_{C-Quelle verbraucht}) | 0,141 | 0,143 | 0,146 | 0,140 | 0,146 | 0,145 | 0,148 | 0,147 | 0,146 | 0,145 | 0,147 | 0,148 |
| Ausbeute nach 70,79 h Kultivieru ng (mol_{oAb}/m ol_{C-Quelle vorgelegt}) | 0,141 | 0,143 | 0,146 | 0,140 | 0,146 | 0,145 | 0,148 | 0,147 | 0,146 | 0,145 | 0,147 | 0,146 |

### Finale Produktausbeute

**Tabelle 59: Übersicht über die ortho-Aminobenzoesäureausbeuten nach 70,79 h für sechs Replikate mit Medium 3 und Medium 4. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Bedingung (Gew.-% Zucker bezogen auf Gesamtzucker) | Medium 3 (76 Gew.-% _{D}-Glucose, 24 Gew.-% _{D}-Xylose) | | | | | | Medium 4 (63 Gew.-% _{D}-Glucose, 37 Gew.-% _{D}-Xylose) | | | | | |
|---|---|---|---|---|---|---|---|---|---|---|---|---|
| Position in MTP | B5 | B6 | B7 | B8 | C1 | C2 | C3 | C4 | C5 | C6 | C7 | C8 |
| Ausbeute nach 70,79 h Kultivierung (go_{Ab}/g_{C-Quelle} verbraucht) | 0,120 | 0,123 | 0,121 | 0,122 | 0,121 | 0,121 | 0,124 | 0,123 | 0,127 | 0,125 | 0,127 | 0,129 |
| Ausbeute nach 70,79 h Kultivierung (go_{Ab}/g_{C-Quelle} vorgelegt) | 0,118 | 0,120 | 0,118 | 0,119 | 0,118 | 0,116 | 0,113 | 0,113 | 0,114 | 0,112 | 0,114 | 0,114 |
| Ausbeute nach 70,79 h Kultivierung (mol_{oAb}/mol_{C-Quelle verbraucht}) | 0,151 | 0,155 | 0,152 | 0,153 | 0,152 | 0,152 | 0,154 | 0,153 | 0,157 | 0,156 | 0,158 | 0,160 |
| Ausbeute nach 70,79 h Kultivierung (mol_{oAb}/mol_{C-quelle vorgelegt}) | 0,147 | 0,150 | 0,148 | 0,149 | 0,148 | 0,146 | 0,139 | 0,139 | 0,139 | 0,137 | 0,140 | 0,139 |

### Finale Produktausbeute

**Tabelle 60: Übersicht über die ortho-Aminobenzoesäureausbeuten nach 70,79 h für sechs Replikate mit Medium 5 und Medium 6. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Bedingung (Gew.-% Zucker bezogen auf Gesamtzucker) | Medium 5 (50 Gew.-% _{D}-Glucose, 50 Gew.-% _{D}-Xylose) | | | | | | Medium 6 (39 Gew.-% _{D}-Glucose, 61 Gew.-% _{D}-Xylose) | | | | | |
|---|---|---|---|---|---|---|---|---|---|---|---|---|
| Position in MTP | D1 | D2 | D3 | D4 | D5 | D6 | D7 | D8 | E1 | E2 | E3 | E4 |
| Ausbeute nach 70,79 h Kultivierung (g_{oAb}/g_{C-Quelle} verbraucht) | 0,129 | 0,129 | 0,128 | 0,126 | 0,128 | 0,132 | 0,139 | 0,138 | 0,139 | 0,139 | 0,136 | 0,140 |
| Ausbeute nach 70,79 h Kultivierung (g_{oAb}/g_{C-Quelle} vorgelegt) | 0,102 | 0,101 | 0,101 | 0,100 | 0,100 | 0,103 | 0,089 | 0,087 | 0,088 | 0,087 | 0,085 | 0,086 |
| Ausbeute nach 70,79 h Kultivierung (mol_{oAb}/mol_{C-Quelle verbraucht}) | 0,158 | 0,158 | 0,157 | 0,154 | 0,157 | 0,162 | 0,169 | 0,168 | 0,170 | 0,169 | 0,167 | 0,172 |
| Ausbeute nach 70,79 h Kultivierung (mol_{oAb}/mol_{C-Quelle vorgelegt}) | 0,121 | 0,120 | 0,120 | 0,119 | 0,119 | 0,122 | 0,104 | 0,102 | 0,104 | 0,102 | 0,100 | 0,100 |

### Finale Produktausbeute

**Tabelle 61: Übersicht über die ortho-Aminobenzoesäureausbeuten nach 70,79 h für sechs Replikate mit Medium 7 und Medium 8. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Bedingung (Gew.-% Zucker bezogen auf Gesamtzucker) | Medium 7 (27 Gew.-% _{D}-Glucose, 73 Gew.-% _{D}-Xylose) | | | | | | Medium 8 (14 Gew.-% _{D}-Glucose, 86 Gew.-% _{D}-Xylose) | | | | | |
|---|---|---|---|---|---|---|---|---|---|---|---|---|
| Position in MTP | E5 | E6 | E7 | E8 | F1 | F2 | F3 | F4 | F5 | F6 | F7 | F8 |
| Ausbeute nach 70,79 h Kultivierung (g_{oAb}/g_{C-Quelle} verbraucht) | 0,147 | 0,147 | 0,148 | 0,148 | 0,154 | 0,147 | 0,173 | 0,170 | 0,168 | 0,169 | 0,164 | 0,198 |
| Ausbeute nach 70,79 h Kultivierung (g_{oAb}/g_{C-Quelle vorgelegt}) | 0,066 | 0,067 | 0,067 | 0,067 | 0,070 | 0,071 | 0,045 | 0,041 | 0,042 | 0,041 | 0,040 | 0,040 |
| Ausbeute nach 70,79 h Kultivierung (mol_{oAb}/mol_{C-Quelle verbraucht}) | 0,178 | 0,178 | 0,179 | 0,180 | 0,187 | 0,178 | 0,208 | 0,205 | 0,203 | 0,204 | 0,198 | 0,244 |
| Ausbeute nach 70,79 h Kultivierung (mol_{oAb}/mol_{C-Quelle vorgelegt}) | 0,076 | 0,077 | 0,077 | 0,077 | 0,080 | 0,081 | 0,051 | 0,046 | 0,047 | 0,046 | 0,045 | 0,045 |

### Mittelwerte die finalen Produktausbeuten und Standardabweichungen

**Tabelle 62: Übersicht über die Mittelwerte und Standardabweichungen der ortho-Aminobenzoesäureausbeuten aus je sechs Replikaten nach 70,79 h. Diese werden angegeben als massenbezogener (g_{oAb}/g_{C-Quelle}) und stoffmengenbezogener Quotient (mol_{oAb}/mol_{C-Quelle}), wobei zum einen die metabolisierte und zum anderen die im Medium vorgelegte Kohlenstoffquelle in die Rechnung einbezogen wird.**

| Medium Nr. (-) | Medium 1 | Medium 2 | Medium 3 | Medium 4 | Medium 5 | Medium 6 | Medium 7 | Medium 8 |
|---|---|---|---|---|---|---|---|---|
| Bedingung (Gew.-% Zucker bezogen auf Gesamtzucker) | 100 Gew.-% _{D}-Glucose, 0 Gew.-% _{D}-Xylose | 86 Gew.-% _{D}-Glucose, 14 Gew.-% _{D}-Xylose | 76 Gew.-% _{D}-Glucose, 24 Gew.-% _{D}-Xylose | 63 Gew.-% _{D}-Glucose, 37 Gew.-% _{D}-Xylose | 50 Gew.-% _{D}-Glucose, 50 Gew.-% _{D}-Xylose | 39 Gew.-% _{D}-Glucose, 61 Gew.-% _{D}-Xylose | 27 Gew.-% _{D}-Glucose, 73 Gew.-% _{D}-Xylose | 14 Gew.-% _{D}-Glucose, 86 Gew.-% _{D}-Xylose |
| Mittelwert der Ausbeute nach 70,79 h Kultivierung (g_{oAb}/g_{C-Quelle} verbraucht) | 0,109 ± 0,002 | 0,115 ± 0,001 | 0,121 ± 0,001 | 0,126 ± 0,002 | 0,129 ± 0,002 | 0,139 ± 0,001 | 0,149 ± 0,003 | 0,174 ± 0,012 |
| Mittelwert der Ausbeute nach 70,79 h Kultivierung (g_{oAb}/g_{C-Quelle} vorgelegt) | 0,109 ± 0,002 | 0,115 ± 0,001 | 0,118 ± 0,001 | 0,113 ± 0,001 | 0,101 ± 0,001 | 0,087 ± 0,001 | 0,068 ± 0,002 | 0,042 ± 0,002 |
| Mittelwert der Ausbeute nach 70,79 h Kultivierung (mol_{oAb}/mol_{C-Quelle verbraucht}) | 0,144 ± 0,003 | 0,147 ± 0,001 | 0,153 ± 0,001 | 0,156 ± 0,003 | 0,158 ± 0,003 | 0,169 ± 0,002 | 0,180 ± 0,003 | 0,210 ± 0,017 |
| Mittelwert der Ausbeute nach 70,79 h Kultivierung (mol_{oAb}/mol_{C-Quelle vorgelegt}) | 0,144 ± 0,003 | 0,146 ± 0,001 | 0,148 ± 0,001 | 0,139 ± 0,001 | 0,121 ± 0,001 | 0,102 ± 0,002 | 0,078 ± 0,002 | 0,047 ± 0,002 |

### Zusammenfassung

In der hier beschriebenen Kultivierung eines xyloseverstoffwechselnden ortho-Aminobenzoesäureproduzenten basierend auf *C*. *glutamicum* im Mikrotiterplattenformat wurde der Einfluss verschiedener Mischungsverhältnisse von Glucose und Xylose auf die Ausbeute von ortho-Aminobenzoesäure in einem erweiterten Mischungsbereich untersucht.

Bis zur Beendigung der Kultivierungen wurde die Xylose in den meisten Fällen noch nicht vollständig verbraucht, weshalb bei der finalen Produktausbeute zwischen der "Substratausbeute" bezogen auf die Masse bzw. Stoffmenge des verbrauchten Substrats (g_{oAb} / g_{verbrauchte C-Quelle} bzw.mol_{oAb} / mol_{verbrauchte C-Quelle}) und der "Prozessausbeute" bezogen auf die Masse bzw. Stoffmenge des vorgelegten Substrats (g_{oAb} / g_{vorgelegte C-Quelle} bzw.mol_{oAb} / mol_{vorgelegte C-Quelle}) unterschieden wurde.

Es wurde festgestellt, dass die metabolische Ausbeute mit zunehmendem Anteil an Xylose stieg. Bei der Prozessausbeute wurde ein Maximum von 0,153 g_{oAb} / g_{vorgelegte C-Quelle} bzw. 0,148 mol_{oAb} / mol_{vorgelegte C-Quelle} mit Medium 3 erreicht, in dem die eingesetzte Energie- und Kohlenstoffquelle zu 76 Gew.-% aus Glukose und 24 Gew.-% Xylose zusammengesetzt war.

In dieser Studie wurde somit überraschend gefunden, dass die Kombination von _{D}-Glucose und _{D}-Xylose in Gemischen mit einem Glucoseanteil von 64-100 Gew.-% und einem Xyloseanteil zwischen 0-36 Gew.-% zu einer effizienteren ortho-Aminobenzoesäure-Produktbildung führt, als die reine Verwendung von _{D}

## Patentansprüche

1. Verfahren, enthaltend den Schritt der Kultivierung einer oder mehrerer mikrobieller Zellen, die Glucose und Xylose zu oAB umsetzen können, in einem Nährmedium, das ein Gemisch von Glucose und Xylose mit einem Glucoseanteil zwischen 5 Gew.-% und 86 Gew.-% und einem Xyloseanteil zwischen 95 Gew.-% und 14 Gew.-% enthält, wobei sich die Anteile von Glucose und Xylose zu 100 % addieren und wobei oAB produziert wird.

2. Das Verfahren gemäß Anspruch 1, wobei das Gemisch von Glucose und Xylose einen Glucoseanteil zwischen 16 Gew.-% und 86 Gew.-% hat und Xylose den zu 100 Gew.-% fehlenden Anteil beiträgt.

3. Das Verfahren gemäß Anspruch 2 oder 3, wobei die Kohlenstoffausbeute wenigstens 0,138 mol Kohlenstoff in Form von oAB pro verbrauchtem mol Kohlenstoff in Glucose und Xylose beträgt.

4. Verfahren gemäß eines der Ansprüche 1 bis 3, **dadurch gekennzeichnet, dass** die mikrobiellen Zellen die in Anspruch 5 definierten genetischen Modifikationen aufweisen.

5. Zelle des Genus *Corynebacterium,* die vom Wildtyp wenigstens in folgenden Merkmalen abweicht:
(i) Gegenüber dem Wildtyp reduzierte Expression der Anthranilat-Phosphoribosyltransferase, wobei aber eine Restaktivät vorhanden sein muss;
(ii) Inaktivierung oder wenigstens teilweise des Gens, das für die Phophoenolpyruvat-Carboxylase (ppc) kodiert;
(iii) Erhöhte Aktivität der Shikimat-Kinase;
(iv) Erhöhte Aktivtät der 3-Phosphoshikimat 1-carboxyvinyltransferase und Chorismat-Synthase;
(v) Anwesenheit einer feedback-resistenten 3-Desoxyarabinoheptulosanat-7-phosphat-Synthase; und
(vi) Erhöhte Aktivität der Xylose-Isomerase und der Xylulokinase.

6. Verwendung eines Stamms des Genus *Corynebacterium* gemäß Anspruch 5 zur Herstellung von oAB durch eine mikrobielle Fermentation mit einem Gemisch von Glucose und Xylose als Energie- und Kohlenstoffquelle.

7. Verwendung gemäß Anspruch 6, wobei das Gemisch von Glucose und Xylose einen Glucoseanteil zwischen 5 Gew.-% und 86 Gew.-% und einem Xyloseanteil zwischen 95 Gew.-% und 14 Gew.-% enthält und wobei sich die Anteile von Glucose und Xylose zu 100 % addieren.

8. Verwendung gemäß Anspruch 6 oder 7, **dadurch gekennzeichnet, dass** die mikrobiellen Zellen die in Anspruch 5 definierten genetischen Modifikationen aufweisen.

9. Zusammensetzung enthaltend
a) mikrobielle Zellen, die Glucose und Xylose zu oAB umsetzen können;
b) ein Gemisch von Glucose und Xylose mit einem Glucoseanteil zwischen 5 Gew.-% und 86 Gew.-% und einem Xyloseanteil zwischen 95 Gew.-% und 14 Gew.-%, wobei sich die Anteile von Glucose und Xylose zu 100 % addieren; und
c) wenigstens eine Stickstoffquelle, wenigstens eine Phosphorquelle, wenigstens eine Schwefelquelle und Spurenelemente.

10. Zusammensetzung gemäß Anspruch 9, zusätzlich enthaltend oAB.

11. Zusammensetzung gemäß Anspruch 9 oder 10, **dadurch gekennzeichnet, dass** die mikrobiellen Zellen die in Anspruch 5 definierten genetischen Modifikationen aufweisen.

12. Verwendung eines Nährmediums, das ein Gemisch von Glucose und Xylose mit einem Glucoseanteil zwischen 5 Gew.-% und 86 Gew.-% und einem Xyloseanteil zwischen 95 Gew.-% und 14 Gew.-% enthält, wobei sich die Anteile von Glucose und Xylose zu 100 % addieren, zur Herstellung von oAB durch eine mikrobielle Fermentation
